# EUROPEAN PATENT APPLICATION

(11) **EP 3 553 061 A1**
(43) Date of publication of application: **16.10.2019**
(21) Application number: 18305453.5
(22) Date of filing: 12.04.2018
(51) Int. Cl.: C07D 487/10, A61P 19/08, A61K 31/429, C07D 513/10

(54) **NEW INHIBITORS OF BONE RESORPTION**

(71) Applicant: CENTRE NATIONAL DE LA RECHERCHE SCIENTIFIQUE (CNRS), 75016 Paris (FR); UNIVERSITE DE MONTPELLIER, 34090 Montpellier (FR); Axlr, Satt Du Languedoc Roussillon, 34090 Montpellier (FR)
(72) Inventor: BLANGY-BLOT, Anne, 34000 MONTPELLIER (FR); MOUNIER, Lucile, 34090 MONTPELLIER (FR)
(74) Representative: Regimbeau

(57) **Abstract**

The invention relates to a compound of formula (I): 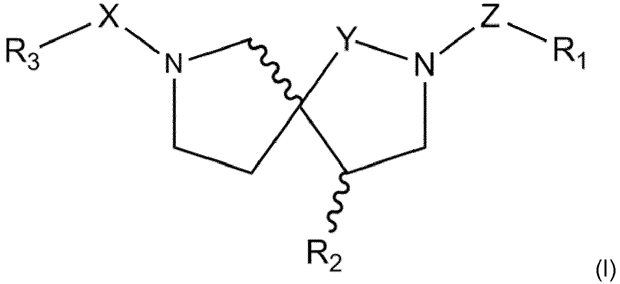 and its use as drug, in particular for use in prevention and/or treatment of disease-associated bone loss, preferably selected in the group consisting of bone metastases, multiple myeloma, osteoporosis, osteopenia due to bone metastases, osteogenesis imperfecta, periarticular erosions in rheumatoid arthritis, primary hyperparathyroidism, hypercalcemia of malignancy, Paget's disease of bone, periodontal disease, immobilization induced osteopenia, and glucocorticoid treatment.

## Description

### FIELD OF THE INVENTION

The invention relates to the identification of new chemical compounds that inhibit bone resorption by osteoclasts. More precisely these molecules inhibit the activation of GTPase Rac by exchange factor Dock5. This activity is necessary to organize the osteoclast's podosomes into a belt, so that it can resorb the bone.

These chemical compounds represent a new class of compounds inhibiting bone resorption paving the way towards the development of new drugs for the treatment of/or prevention of bone loss in osteolytic diseases as postmenopausal osteoporosis, bone metastases, multiple myeloma, rheumatoid arthritis, and various bone genetic diseases.

### BACKGROUND OF THE INVENTION

Bone is a dynamic tissue that is continually remodeled throughout life depending on factors such as nutrition and the load the bone must carry. Normal bone formation depends on the delicate balance between new bone addition and old bone resorption. Bone formation is based on the deposition of bone matrix by osteoblasts and bone resorption and more specifically mineralized tissue, chiefly calcium carbonate and calcium phosphate resorption in vertebrates is achieved by osteoclasts. Typically, in a normal adult, about 5-10% of bone is replaced by these processes annually.

These osteoclasts are multinucleated cells of up to 400µm related to macrophage and other cells that develop from monocyte cells, which are actively motile cells that migrate along the surface of bone. Like macrophage, osteoclasts are derived from haematopoietic progenitor cells. The bone resorption is initiated when an osteoclast attaches to the surface of mineralized bone, forms a tight "sealing zone" and secretes necessary acids and proteases that initiate the resorption of mineralized tissue from the bone. After a period of several hours to days, the osteoclast detaches from the bone, leaving a pit on the bone surface. Under normal conditions, the pit is a target for osteoblasts, which deposit a material that ultimately becomes new bone.

Bone loss can appear when the bone resorptive process is dominant over the bone formative process. Diseases associated with bone loss are usually accompanied by increased osteoclast activation. Such diseases include any bone loss resulting notably from an estrogen deficiency after the menopause but not only and comprise osteoporosis, osteopenia due to bone metastases, periarticular erosions in rheumatoid arthritis, primary hyperparathyroidism, hypercalcemia of malignancy, Paget's disease of bone, periodontal disease, immobilization induced osteopenia, and glucocorticoid treatment.

The available treatments of osteolytic diseases are not effective in all patients, and produce adverse side effects.
Treatments used nowadays to limit the bone loss due to the exacerbated activity of osteoclasts are mainly molecules from nitrogen bisphosphonate family. By preventing bone resorption by osteoclasts, bisphosphonates are medications that prevent the skeleton embrittlement and the establishment of osteoporosis. Bisphosphonates help to protect the bones against the effects of certain cancers and to treat some bone disorders such as postmenopausal osteoporosis or rheumatoid arthritis. For the osteoporosis treatment, bisphosphonates of reference are the zoledronate (Aclasta, Zometa), the alendronate (Fosamax), the risedronate (Actonel) or the etidronate (Didrocal, Didronel). Bisphosphonates cause osteoclast death by apoptosis. The Denosumab, a monoclonal antibody directed against the cytokine RANKL, has recently been placed on the market (Prolia, Xgeva). It prevents the differentiation of osteoclasts.
In the two cases, the treatment results in the disappearance of the osteoclasts. These having an action that stimulates bone formation by osteoblasts, they cause a secondary loss of bone formation (Cappariello, 2014). Bones are stuck. This leads to a problem of response to the bone anabolic action of the parathyroid hormone. Furthermore, the lack of bone dynamics is suspected of participating in the onset of jaw osteonecrosis and also atypical fractures, the incidence of which is increasing. The proposed approach, which targets the osteoclast activity but not its differentiation or survival, makes it possible to overcome this problem.

We previously demonstrated that bone resorption by osteoclasts depends on the activation of GTPase Rac by Dock5. Dock5 allows the restructuring of the actin cytoskeleton of the osteoclast so that it produces in contact with the bone a structure allowing an acid attack to solubilize the mineral and a proteolytic attack to digest the collagen. Dock5 is only necessary to the bone resorption, not to the differentiation or to the osteoclasts survival. Furthermore, its suppression in the mouse does not lead to a particular phenotype, apart from the increase of bone mass, with a normal number of osteoclasts (Vives et al, 2011). We also demonstrated that the inhibitors of Dock5 can prevent bone resorption by osteoclasts in culture and *in vivo* in mice. The principle of identification of inhibitors of bone resorption by targeting the activation of GTPase Rac by Dock5 is disclosed in the patent application WO2010/020647, with examples of commercial molecules active in culture. An inhibitor of Dock5 the N-(3,5-dichlorophenyl)-benzenesulfonamide, called C21 (Vives et al., 2011), has been used in the mouse in three classical models of bone loss: postmenopausal osteoporosis, inflammatory bone loss, and metastasis-induced bone loss.
Upon intravenous or intraperitoneal administration, C21 inhibits effectively the bone resorption in these three models. C21 also reduces the development of bone metastases, an expected effect of inhibition of osteoclast activity (US2013/0165523). Bone formation is maintained in mice treated with C21 daily along 4 weeks, unlike the mice treated with bisphosphonate (Alendronate). Histological observation of all tissues of the mice did not reveal any anomaly and these results were published (Vives et al., 2015).

But there is still a need to provide new compounds efficient on human osteoclasts, in particular on inhibition of the activation of Rac by Dock5, with no secondary loss of bone formation, contrary to the available bisphosphonates compounds and Denosumab.
The present invention relates to new chemical compounds that inhibit bone resorption by osteoclasts. More precisely these molecules inhibit the activation of GTPase Rac by exchange factor Dock5. This activity is necessary to organize the osteoclast's podosomes into a belt, so that it can resorb the bone. The present invention describes the activity of these molecules on Rac activation by Dock 5, in a reporter cell system to discriminate active and non-active compounds. These compounds represent a new class of compounds inhibiting bone resorption without affecting bone formation paving the way towards the development of new drugs for the treatment of/or prevention of bone loss in osteolytic diseases as postmenopausal osteoporosis, bone metastases, multiple myeloma, rheumatoid arthritis, and various bone genetic diseases.

### SUMMARY OF THE INVENTION

A first object of the invention is a compound of formula (I): 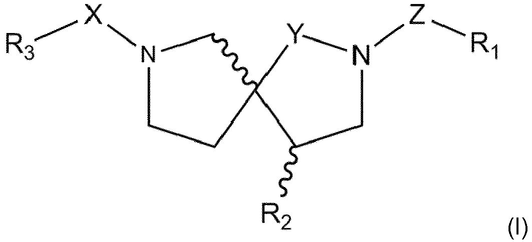 or a pharmaceutically acceptable salt thereof or solvate or stereoisomer or a mixture of stereoisomers, wherein
X is -CH₂-, -CH₂-CH₂-, -C(O)-, -C(O) -CH₂-, -SO₂-, -C(O)NH-, -OC(O)NH-, -C(O)O-, or a single bond;
Y is -C(O)- or -S(O)₂-;
Z is -CH₂- or a single bond;
R₁ is a saturated, unsaturated or aromatic 5- or 6-membered carbocycle or heterocycle, optionally substituted with one to three substituents independently selected from the group consisting of halogen, C₁-C₆ alkyl, C₁-C₆ alkoxy, NH(C₁-C₆ alkyl), N(C₁-C₆ alkyl)₂, C₁-C₆ alcohol;
R₂ is H or -OR₄; wherein R₄ is a -C₁-C₆ alkyl group optionally substituted by -OH, -OR, - COOH, -COOR, -CONHR or -CONRR' with R and R' independently representing a C₁-C₆ alkyl group; or wherein R₄ is an aromatic 5- to 7-membered carbocycle or heterocycle; R₃ is:
   - H or a C₁-C₆ alkyl group, provided that when R₃ is H, X is not -C(O)- or -SO₂- ; or
   - a saturated or unsaturated 3- to 7-membered carbocycle or heterocycle, optionally substituted with one to three substituents independently selected from the group consisting of halogen, C₁-C₆ alkyl and C₁-C₆ alkoxy group; or
   - aromatic 5- or 10-membered carbocycle or heterocyclean, optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, and a C₁-C₆ alkyl wherein 1 to 3 hydrogen atoms are replaced by a fluorine atom, S(O)₂R, COOH, COOR, CONHR or CONRR' with R and R' independently representing a C₁-C₆ alkyl group.

Another object of the invention is a pharmaceutical composition comprising a compound of formula (I) of the invention as active ingredient, and a pharmaceutically acceptable excipient.

Another object of the invention relates to the compound of formula (I) of the invention or a pharmaceutical composition comprising said compound of formula (I), for use as drug.

Another object of the invention relates to the compound of formula (I) of the invention or a pharmaceutical composition comprising said compound of formula (I), for use in prevention and/or treatment of disorder-associated bone loss or disease-associated bone loss, preferably selected in the group consisting of menopause, osteoporosis, osteopenia due to bone metastasis, osteogenesis imperfecta, inflammatory arthritis, particularly rheumatoid arthritis, more particularly periarticular erosions in rheumatoid arthritis, primary hyperparathyroidism, hypercalcemia of malignancy, Paget's disease of bone, periodontal disease, immobilization induced osteopenia, and glucocorticoid treatment; or prevention and/or treatment of bone loss caused by cancer, particularly multiple myeloma and bone metastases, particularly bone metastases associated with cancer.

### DETAILED DESCRIPTION OF THE INVENTION

### Definitions

For the purpose of the invention, the term "pharmaceutically acceptable" is intended to mean what is useful to the preparation of a pharmaceutical composition, and what is generally safe and non-toxic, for a pharmaceutical use.
The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide. Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The terms "C₁-C₆ alkyl group", as used in the present invention, refers to a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, iso-pentyl, sec-pentyl, *tert*-pentyl, n-hexyl, iso-hexyl, *sec*-hexyl, *tert*-hexyl, and the like.

The term "C₁-C₆ alkoxy", as used in the present invention, refers to a (C₁-C₆)alkyl group as defined above bound to the molecule via an oxygen atom, including, but not limited to, methoxy, ethoxy, n-propoxy, iso-propoxy, n-butoxy, iso-butoxy, sec-butoxy, t-butoxy, n-pentoxy, n-hexoxy, and the like.

The term "carbocycle", as used in the present invention, refers to a saturated, unsaturated or aromatic hydrocarbon monocycle or polycycle (comprising fused, bridged or spiro rings), such as a bicycle. A carbocycle can be notably, but not limited to, a cyclopropyl, cyclopentyl, cyclohexyl, cycloheptyl, phenyl or naphtyl group.

The term "5- to 10-membered carbocycle" as used in the present invention, refers to a carbocycle as defined above having 5 to 10 atoms in the ring(s).

The term "3- to 7-membered carbocycle" as used in the present invention, refers to a carbocycle as defined above having 3 to 7 atoms in the ring(s).

The term "5- or 6-membered carbocycle" as used in the present invention, refers to a carbocycle as defined above having 5 or 6 atoms in the ring(s).

The term "heterocycle" as used in the present invention refers to a saturated, unsaturated or aromatic hydrocarbon monocycle or polycycle (comprising fused, bridged or spiro rings), such as a bicycle, in which one or more, advantageously 1 to 4, and more advantageously 1 or 2, carbon atoms have each been replaced with a heteroatom selected from nitrogen, oxygen and sulphur atoms. Advantageously, the heterocycle comprises 3 to 15, notably 3 to 10 atoms in the ring(s). A heterocycle can be notably thiophene, furan, pyrrole, imidazole, pyrazole, oxazole, isoxazole, thiazole, isothiazole, isothiazolidine, triazoles (1,2,3-triazole and 1,2,4-triazole), benzofuran, indole, benzothiophene, benzimidazole, indazole, benzoxazole, benzisoxazole, benzothiazole, benzisothiazole, pyridine, pyrimidine, pyridazine, pyrazine, triazine, quinoline, isoquinoline, quinoxaline, quinazoline, piperidine, piperazine, triazinane, morpholine, pyrrolidine, dihydropyridines, dihydropyrimidines (notably 1,2-dihydropyrimidine), dihydropyridazines, dihydropyrazines, dihydrotriazines, tetrahydropyridines, tetrahydropyrimidines, tetrahydropyridazines, tetrahydropyrazines, tetrahydrotriazines, tetrahydrofuran, dioxane, dioxalane, etc. In particular, the heterocycle is piperidine or piperazine.

The term "5- to 10-membered heterocycle" as used in the present invention, refers to a heterocycle as defined above having 5 to 10 atoms in the ring(s).

The term "3- to 7-membered heterocycle" as used in the present invention, refers to a heterocycle as defined above having 3 to 7 atoms in the ring(s).

The term "5- or 6-membered heterocycle" as used in the present invention, refers to a heterocycle as defined above having 5 or 6 atoms in the ring(s).

The term "halogen" or "halo", as used in the present invention, refers to a fluorine, bromine, chlorine or iodine atom.

Within the meaning of this invention, "stereoisomers" is intended to designate diastereoisomers or enantiomers. These are therefore optical isomers. Stereoisomers which are not mirror images of one another are thus designated as "diastereoisomers," and stereoisomers which are non-superimposable mirror images are designated as "enantiomers".

Notably, the sugar moiety and the amino acid moieties of the compounds of the invention can belong to the D or L series.

A carbon atom bond to four non-identical substituents is called a "chiral centre".

An equimolar mixture of two enantiomers is called a racemate mixture.

### Compounds of formula (I)

A first object of the invention is a compound of formula (I): 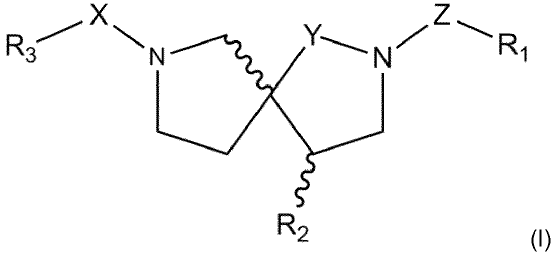 or a pharmaceutically acceptable salt thereof or solvate or stereoisomer or a mixture of stereoisomers, wherein
X is -CH₂-, -CH₂-CH₂-, -C(O)-, -C(O)-CH₂-, -SO₂-, -C(O)NH-, -OC(O)NH-, -C(O)O-, or a single bond;
Y is -C(O)- or -S(O)₂-;
Z is -CH₂- or a single bond;
R₁ is a saturated, unsaturated or aromatic 5- or 6-membered carbocycle or heterocycle, optionally substituted with one to three substituents independently selected from the group consisting of halogen, C₁-C₆ alkyl, C₁-C₆ alkoxy, -NH(C₁-C₆ alkyl), -N(C₁-C₆ alkyl)₂, C₁-C₆ alcohol;
R₂ is H or -OR₄; wherein R₄ is a -C₁-C₆ alkyl group optionally substituted by -OH, -OR, - COOH, -COOR, -CONHR or -CONRR' with R and R' independently representing a C₁-C₆ alkyl group; or wherein R₄ is an aromatic 5- to 7-membered carbocycle or heterocycle;
R₃ is:
   - H or a C₁-C₆ alkyl group, provided that when R₃ is H, X is not -C(O)- or -SO₂- ; or
   - a saturated or unsaturated 3- to 7-membered carbocycle or heterocycle, optionally substituted with one to three substituents independently selected from the group consisting of halogen, C₁-C₆ alkyl and C₁-C₆ alkoxy group; or
   - aromatic 5- or 10-membered carbocycle or heterocyclean, optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, and a C₁-C₆ alkyl wherein 1 to 3 hydrogen atoms are replaced by a fluorine atom, S(O)₂R, COOH, COOR, CONHR or CONRR' with R and R' independently representing a C₁-C₆ alkyl group.

Advantageously, the compound of formula (I) can have the following structure (Ia) and/or (Ib):

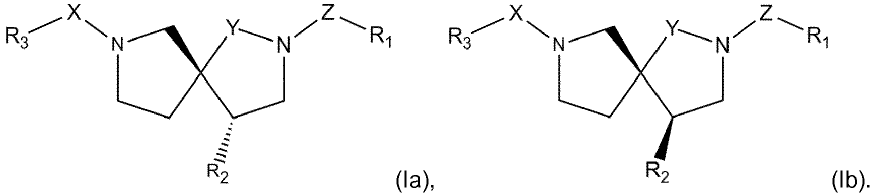

Advantageously, R₁ is a cyclohexyl group or a phenyl group, optionally substituted with one or two substituents independently selected from the group consisting of halogen, C₁-C₆ alkyl, and C₁-C₆ alkoxy group; more advantageously R₁ is cyclohexyl group or a phenyl group, optionally substituted with one or two substituents independently selected from the group consisting of halogen and methoxy; more advantageously R₁ is cyclohexyl, phenyl, 3-chlorophenyl, 3-fluorophenyl or 3-methyl-4-methoxyphenyl, more advantageously R₁ is phenyl.

Advantageously, R₄ is H or a C₁-C₆ alkyl group optionally substituted by -OH, -OR, - COOH, -COOR, -CONHR or -CONRR'; or a aromatic 5- to 7-membered heterocycle; more advantageously R₄ is H or a C₁-C₆ alkyl group optionally substituted by -OH, -OR, - COOH -COOR or -CONHR; or a pyridinyl; more advantageously R₄ is H or a methyl or an ethyl group, such groups being optionally substituted by -OH, -OR, -COOH -COOR or - CONHR; or a pyridinyl; more advantageously R₄ is H; with R and R' independently representing a C₁-C₆ alkyl group. Advantageously, R and R' are independently methyl or ethyl. Advantageously, R₂ is H or OH.

Advantageously, R₃ is
- H or CH₃
- a saturated 5- to 7-membered carbocycle, optionally substituted with one to three substituents independently selected from the group consisting of halogen, C₁-C₆ alkyl and C₁-C₆alkoxy group, or
- a phenyl, naphthyl, indanyl, indenyl, furanyl, pyrrolyl, thienyl, oxazolyl, isoxazolinyl, thiazolyl, isothiazolyl, imidazolyl, pyrazolyl, pyridinyl, pyrimidinyl, pyrazinyl, pyridazinyl, indolyl, thiophenyl, benzofuranyl, benzothiophenyl, 1,3-benzodioxolyl, quinoleyl, and isoquibnoleyl group, each group being optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, -CO₂CH₃, and a C₁-C₆ alkyl wherein 1 to 3 hydrogen atoms are replaced by a fluorine atom.

More advantageously, R₃ is:
- H or CH₃
- a cyclopentyl or cyclohexyl group, or
- a phenyl, naphthyl, indanyl, isoxazolinyl, imidazolyl, pyrazolyl, thienyl, pyridinyl, indolyl, thiophenyl, benzofuranyl, 1,3-benzodioxolyl, or quinoleyl group, preferably a phenyl, indanyl, benzofuranyl, 1,3-benzodioxolyl, or quinoleyl group, each group being optionally substituted with one to three substituents independently selected from the group consisting of: : halogen, cyano, hydroxy, C₁-C₆ alkyl, C₁-C₆ alkoxy group, NH(C₁-C₆ alkyl), N(C₁-C₆ alkyl)₂, C₁-C₆ alcohol, -CO₂CH₃, and a C₁-C₆ alkyl wherein 1 to 3 hydrogen atoms are replaced by a fluorine atom.

More advantageously, R₃ is selected from the group consisting of:

| | | | |
|---|---|---|---|
| 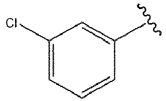 | 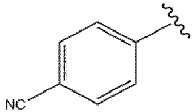 | 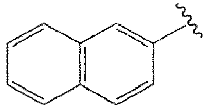 | 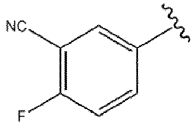 |
| 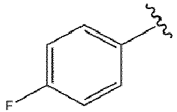 | 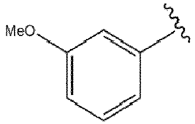 | 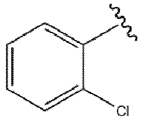 | 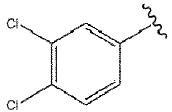 |
| 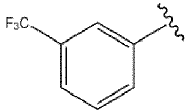 | 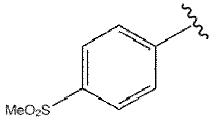 | 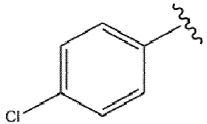 | 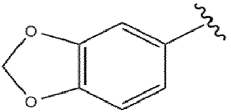 |
| 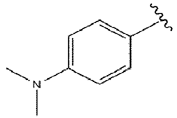 | 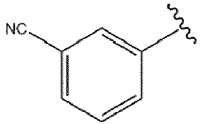 | 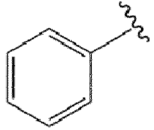 | 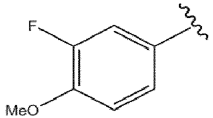 |
| 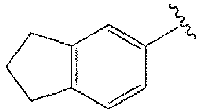 | 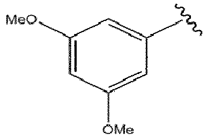 | 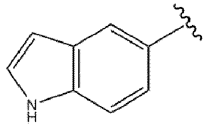 | 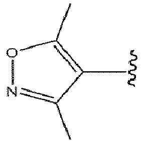 |
| 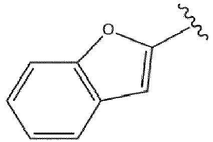 | 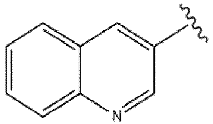 | 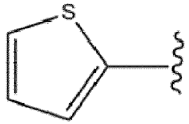 | 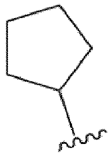 |
| 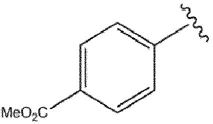 | 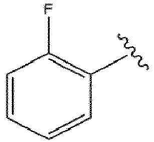 | 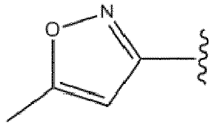 | 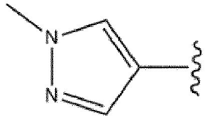 |
| 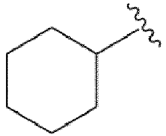 | 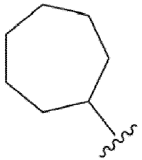 | 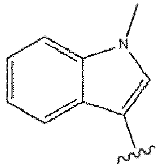 | H |
| CH₃ | | | |

More advantageously, R₃ is selected from the group consisting of:

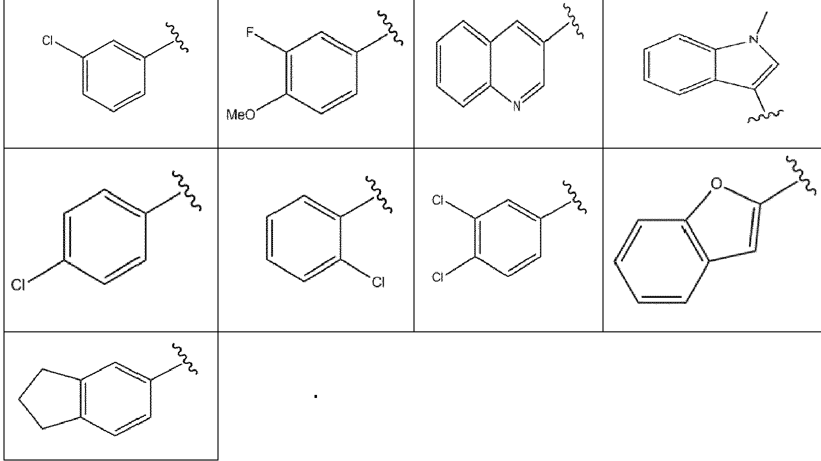

Advantageously, X is -CH₂-, -CH₂-CH₂-, -C(O)-, -C(O)-CH₂-, -SO₂-, or a single bond;
In a particular embodiment, X is CH₂; R₁ is phenyl optionally substituted with one or two substituents independently selected from the group consisting of halogen, methoxy; and R₂ is OH; Y, Z and R₃ being as defined above. Advantageously, X is CH₂; R₁ is phenyl optionally substituted with one or two substituents independently selected from the group consisting of halogen, methoxy; R₂ is OH; and Z is a single bond; Y and R₃ being as defined above, in particular R₃ being as defined in the above-recited tables.

In a particular embodiment, Y is SO₂ and R₂ is -OR₄; X, Z, R₁, R₃ and R₄ being as defined above. Advantageously, Y is SO₂; R₂ is -OR₄; X is CH₂; Z is a single bond; R₁ is phenyl optionally substituted with one or two substituents independently selected from the group consisting of halogen, methoxy; and R₂ is OH; R₃ being as defined above, in particular, R₃ is a phenyl group optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, and a C₁-C₆ alkyl wherein 1 to 3 hydrogen atoms are replaced by a fluorine atom, S(O)₂R, COOH, COOR, CONHR or CONRR' with R and R' independently representing a C₁-C₆ alkyl group.

In a particular embodiment, Y is C(O), X is -CH₂-, Z is a single bond and R₂ is H, R₁ and R₃ being as defined above. Advantageously, Y is C(O); X is -CH₂-; Z is a single bond; R₂ is H; R₁ is phenyl optionally substituted with one or two substituents independently selected from the group consisting of halogen, methoxy; and R₃ is a phenyl group optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, and a C₁-C₆ alkyl wherein 1 to 3 hydrogen atoms are replaced by a fluorine atom, S(O)₂R, COOH, COOR, CONHR or CONRR' with R and R' independently representing a C₁-C₆ alkyl group, in particular R₃ is as defined in the above-recited tables.

In a particular and preferred embodiment, the compound of formula (I) of the invention is selected from the group consisting of:

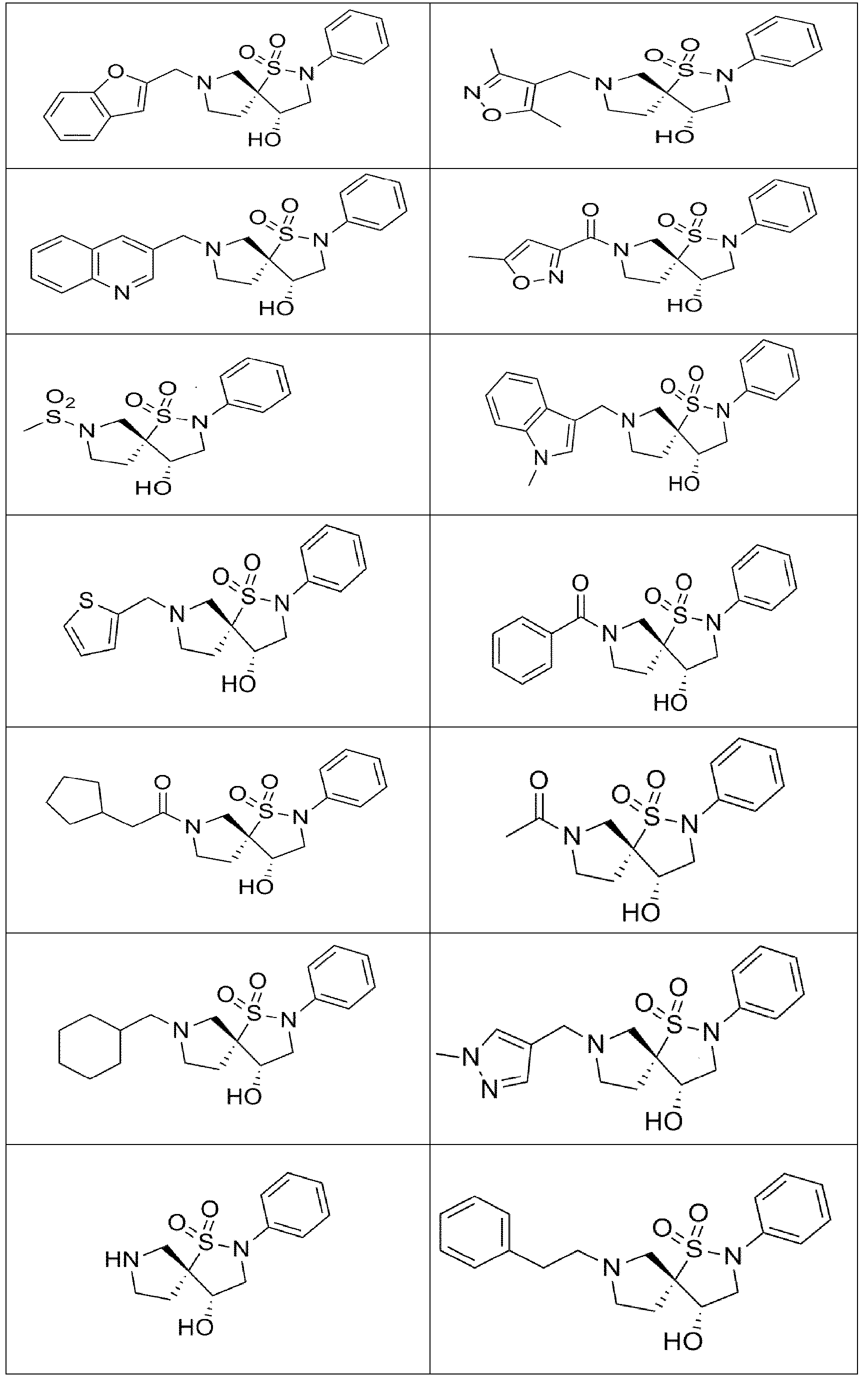

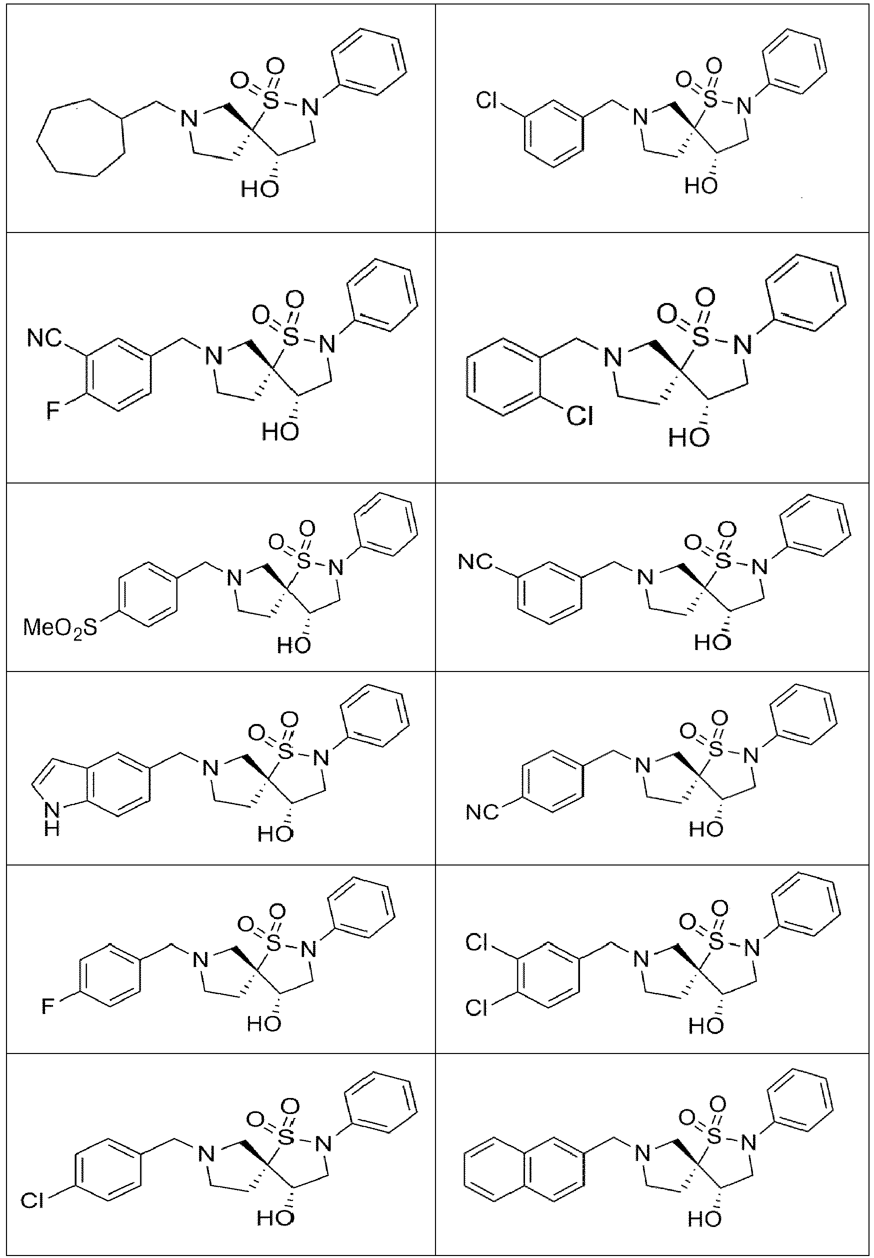

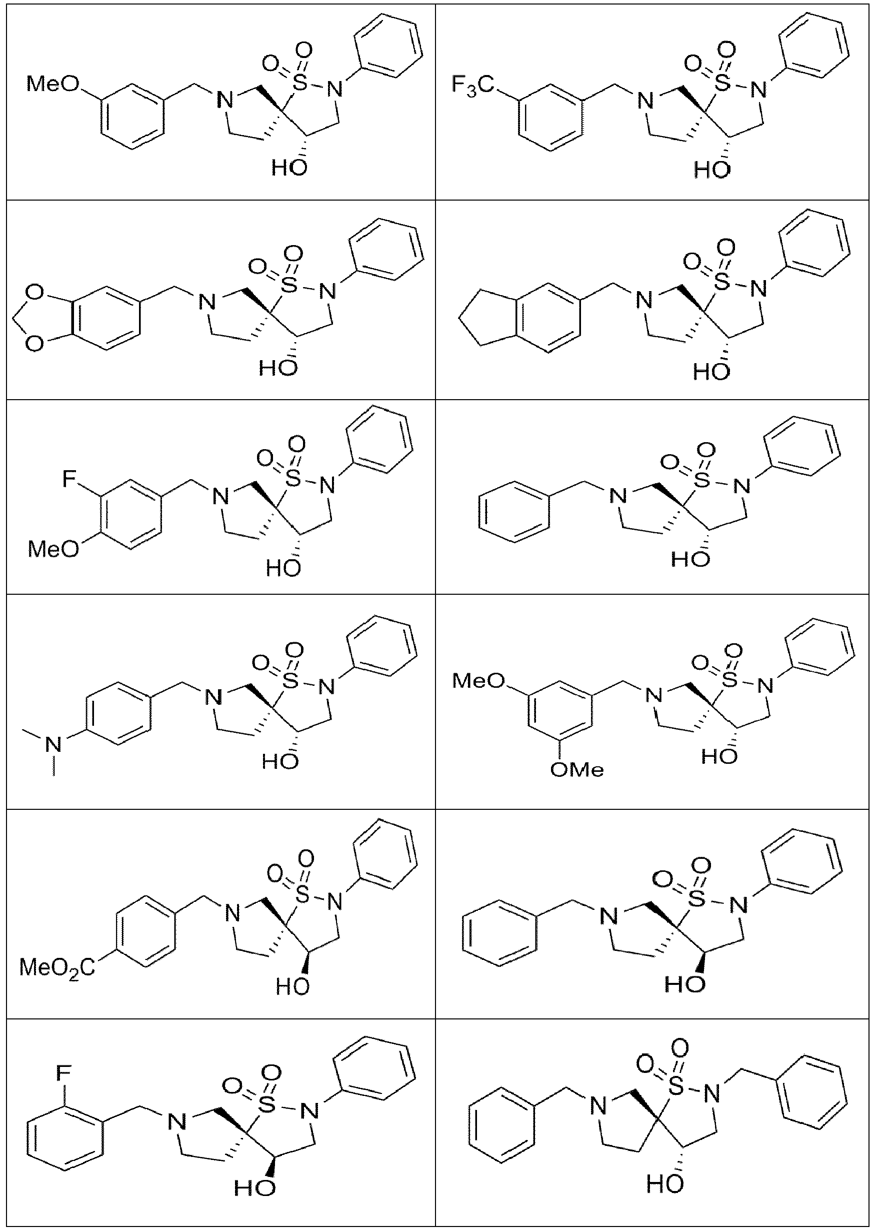

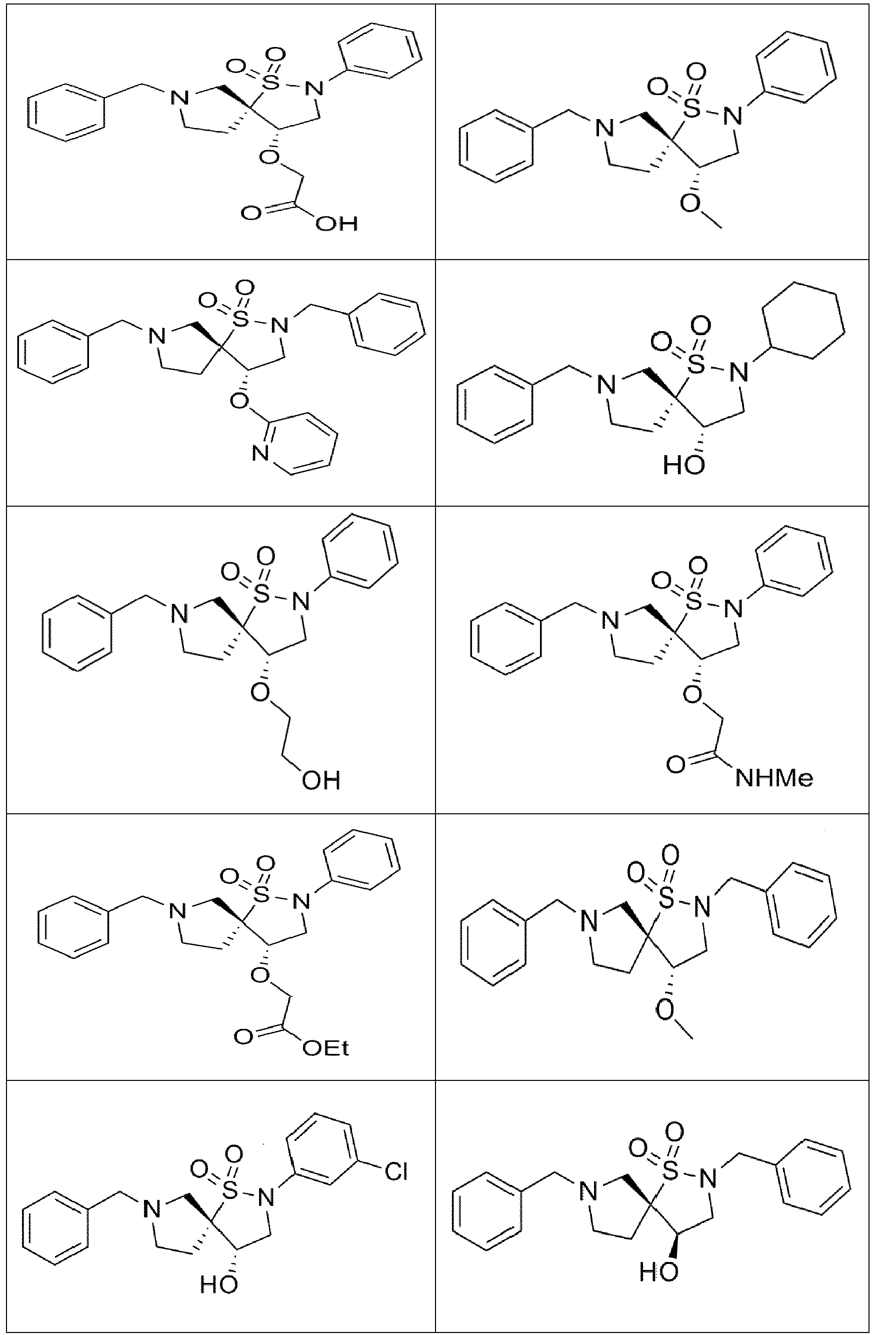

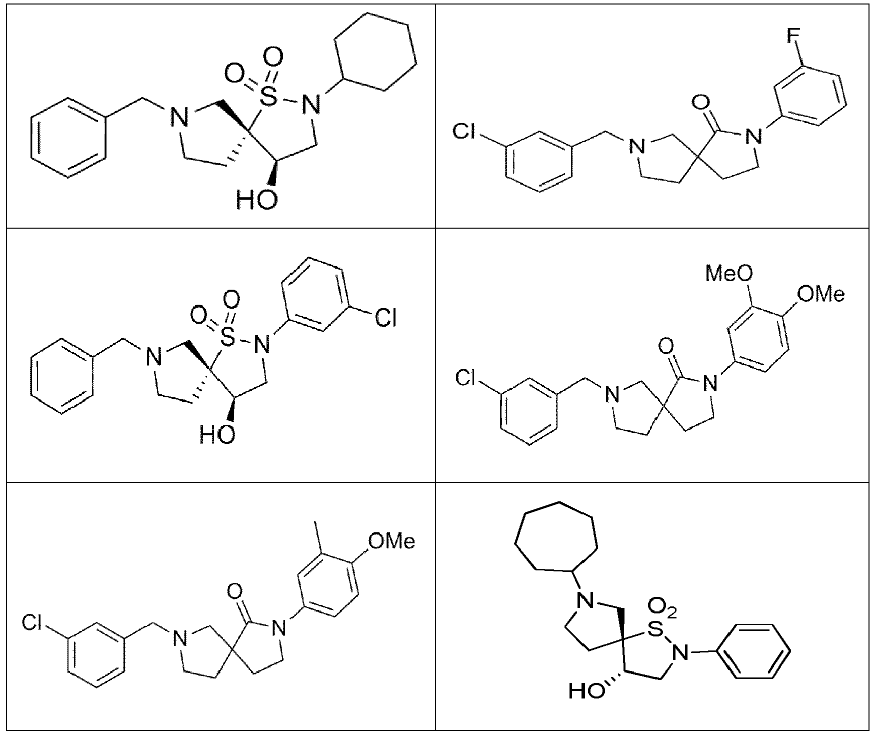

or a pharmaceutically acceptable salt thereof or solvate or stereoisomer or a mixture of stereoisomers.

The compounds of formula (I) of the invention may be obtained by usual chemical synthesis methods known from the man skilled in the art. Advantageously, the compound of formula (I) of the invention can be obtained with a process as described in Example 6 below.

### Pharmaceutical composition

The invention also relates to a pharmaceutical composition comprising a compound of formula (I) of the invention as disclosed above as active ingredient, and a pharmaceutically acceptable excipient.

As examples of pharmaceutically acceptable excipient, the composition can include emulsions, microemulsions, oil in water emulsions, anhydrous lipids and water in oil emulsions or other types of emulsions.

The inventive composition can further include one or more additives such as diluents, excipients, stabilizers and preservatives. Such additives are well known to those skilled in the art and are described notably in *"*Ullmann's Encyclopedia of Industrial Chemistry, 6th Ed." (various editors, 1989-1998, Marcel Dekker) and in *"*Pharmaceutical Dosage Forms and Drug Delivery Systems" (ANSEL et al., 1994 et 2011, WILLIAMS & WILKINS).

The compound of formula (I) of the invention as disclosed above as active ingredient is present in the said pharmaceutical composition in an "effective amount" meaning a quantity that inhibits or reduces the bone resorbing activity of osteoclasts. Those skilled in the art will be able to determine said therapeutically effective quantity based on their general knowledge and on the methods described in the examples.

The compounds can be administered by any mode of administration such as, for example, by intramuscular, intravenous or oral route, etc.
The inventive compounds preferably will be administered at a concentration chosen by those skilled in the art according to the state of advancement of the disease and the targeting mode used, the age and the weight of the subject.
As an example, the compound will be administrated at a concentration between 1 mg/D/kg and 100 mg/D/kg, in particular 2 mg/D/kg and 50 mg/D/kg, or between 5 mg/D/kg and 30 mg/D/kg.

### Uses

Another object of the invention is a compound of formula (I) of the invention or the composition comprising said compound of formula (I), for use as drug.

In a particular embodiment, the invention concerns a compound of formula (I) of the invention or the composition comprising said compound of formula (I), for use in all disorder-associated bone loss or disease-associated bone loss for which bisphosphonates are usually used.

The compounds of the invention are particularly advantageous in comparison to the bisphosphonates which are usually used, as they did not cause a secondary loss of bone formation, contrary to said bisphosphonates compounds.

In a particular embodiment, the invention concerns a compound of formula (I) of the invention or the composition comprising said compound of formula (I), for use the invention concerns a compound of formula (I) of the invention or the composition comprising said compound of formula (I), for use in prevention and/or treatment of disorder-associated bone loss or disease-associated bone loss, preferably selected in the group consisting of menopause, osteoporosis, osteopenia due to bone metastases, osteogenesis imperfecta, inflammatory arthritis, particularly rheumatoid arthritis, more particularly periarticular erosions in rheumatoid arthritis, primary hyperparathyroidism, hypercalcemia of malignancy, Paget's disease of bone, periodontal disease, immobilization induced osteopenia, bone metastasis, particularly bone metastasis associated with breast cancer and glucocorticoid treatment.
More preferably, said disease is osteoporosis.

In another particular embodiment, the invention concerns a compound of formula (I) of the invention or the composition comprising said compound of formula (I), for use in prevention and/or treatment of bone loss caused by cancer, particularly multiple myeloma and bone metastases, particularly bone metastases associated with cancer.
In particular, bone metastasis is associated with cancer, more particularly with a cancer selected in the group consisting of: breast cancer, prostate cancer, lung cancer, colon cancer, stomach cancer and multiple myeloma.

In a particular embodiment, the subject in need thereof is a mammal, in particular an animal or the human.
In a particular embodiment, the subject in need thereof is an animal selected from rodent, cat, dog, primate or equine.
In a particular and preferred embodiment, the subject in need thereof is the human.

### BRIEF DESCRIPTION OF THE FIGURES

Figure 1: Illustration of the screening strategy to identify Dock5 inhibitors.
Figure 2: IC50 of compound E73 on mouse Dock5-DHR2
Figure 3: Effect of the selected molecules on the activity of Dock5 and TRIO in vitro on purified recombinant proteins. Example of molecules: E73, E196, E197, E202, E203, E204, E205, E210, E211, E226, E228.
Figure 4: Effect of the selected molecules on human osteoclasts activity
Figure 5: Trabecular bone volume per tissue volume (BV/TV; %) in femoral metaphysis at the end of in-life phase of the study (at study day 28).
Figure 6: Trabecular bone volume fraction (BV; mm³) in tibial metaphysis at the end of in-life phase of the study (at study day 28).
Figure 7: Trabecular separation (Tb.Sp; µm) in femoral metaphysis at the end of in-life phase of the study (at study day 28).
Figure 8: Trabecular thickness (Tb.Th; µm) in tibial metaphysis at the end of in-life phase of the study (at study day 28).
Figure 9: Cortical thickness (Ct.Th; µm) in tibial diaphysis at the end of in-life phase of the study (at study day 28).
Figure 10: Cortical bone specific surface (BS/BV; mm2/mm3) in tibial diaphysis at the end of in-life phase of the study (at study day 28).
Figure 11: Relative change (%) in serum CTX-I levels during the first two weeks of in-life phase of the study.
Figure 12: Relative change (%) in serum PINP levels during the first two weeks of in-life phase of the study.
Figure 13: Relative change (%) in serum osteocalcin levels during the in-life phase of the study. The relative change was calculated by dividing serum OC levels obtained before the end of the in-life phase (at study day 27) by serum OC levels obtained before the start of the in-life phase (at study day -1).
Figure 14: Mineralizing surface per trabecular bone surface (MS/BS; %) in femoral metaphysis at the end of in-life phase of the study (at study day 28).
Figure 15: Mineral apposition rate (MAR; µm/d) in trabecular bone in femoral metaphysis at the end of in-life phase of the study (at study day 28).
Figure 16: Relative change (%) in body weight during the in-life phase of the study. The relative change was calculated by dividing body weight obtained at the end of the in-life phase (at study day 28) by body weight obtained at the beginning of the in-life phase (at study day 0).

The invention is further illustrated by non-limitative examples.

### EXAMPLES

### Example 1: Identification of the 048ED family.

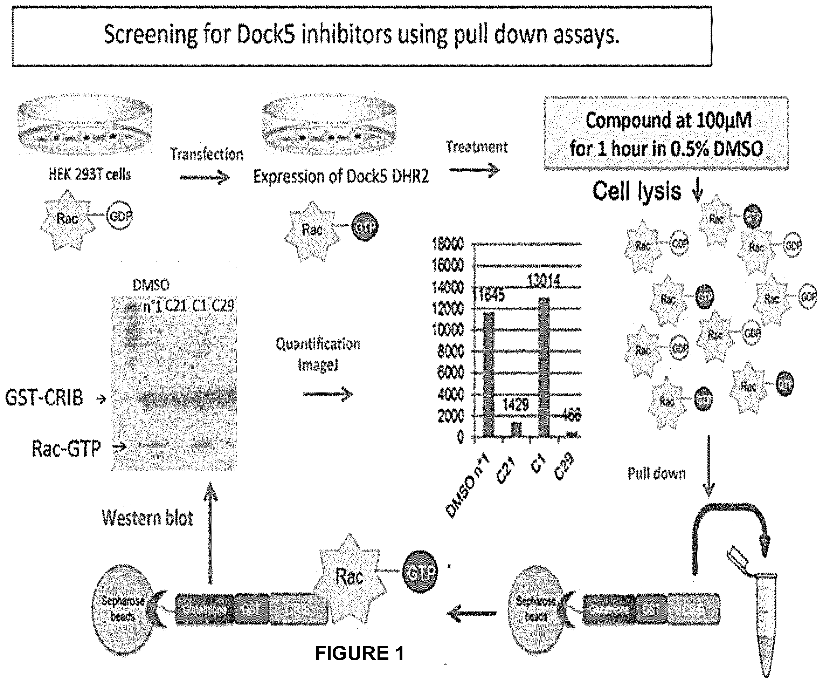

Compounds E73, E88 and E78 were tested for their inhibiting effect of the activation of Rac by mouse Dock5 of and for certain human Dock5 according to a screening test using a pull down assay as represented in Figure 1.

Methodology (Figure 1): cells 293T seeded onto 100 mm diameter plates in a DMEM culture medium containing 10% serum in an incubator in 5.5% CO2 humidified atmosphere at 37°C. When they reach the density of 5x10⁶ cells per plate, cells are transfected with 8 µg of plasmid expressing the catalytic DHR2 domain of mouse or human Dock5 in order to activate endogenous GTPase Rac. After 24 hours, the cells are lifted using trypsin and are seeded 10⁶ per well in 6-well plates of 35 mms in diameter. The following day, the cells are put in contact during one hour with the compound, in a mother solution at 20 mM into 100% DMSO, to a test concentration of 100 µM into 0.5% DMSO in the DMEM medium containing 0.1% of BSA. The basal activation of Rac by Dock5 is measured from the cells treated with 0.5% DMSO. After treatment, the cells are observed under the optical microscope to make sure of the absence of any toxic effect of the compound. If cell suffering is detected, the test is invalidated and the compound is retested at a lower dosage.
The cells are then lysed in 100 µl of the pull down buffer (10% glycerol, 50 mm Tris pH 7.4, 100 mM NaCl, 1% IGEPAL® CA-630 (SIGMA I3021), 2 mM MgCl₂ supplemented with proteases inhibitors) and the lysate is incubated during one hour with 20 µg of sepharose beads coupled with the fusion protein GST-PAK-CRIB, which binds specifically the activated form of GTPase Rac, which is bound to GTP. After centrifugation and washings, the proteins associated with the beads, including active Rac GTPase, are denatured and loaded on an SDS PAGE gel. Rac protein is visualized after a western blot revealed by a luminescent reagent on an autoradiographic film. For each compound tested, the level of active Rac GTPase is quantified using the Image J software and is normalized to the concentration of total protein in the whole cell lysate. The efficiency of the molecule is defined by the level of active Rac after treatment compared to the level of active Rac in the cells treated with DMSO (control). An activity is considered as inhibitory if the level of Rac activation decreases by at least 10%, 20%, in particular at least 30%, 40%, and preferably at least 50% compared to the control treated with DMSO (Table 1). When the level of Rac activation does not decreases, the result is considered as 'negative'.

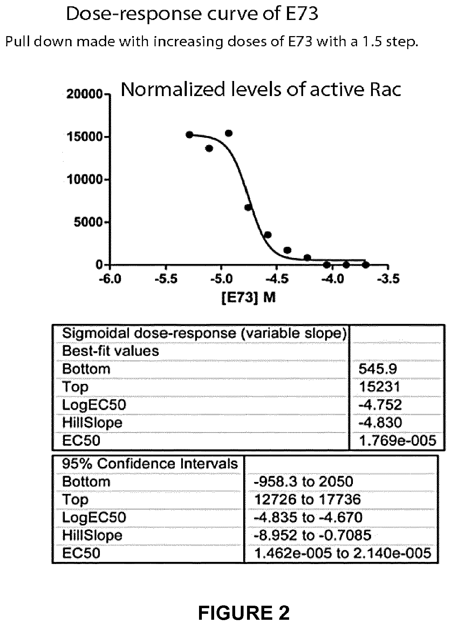

These tests revealed that the compound E73 is the most active on Dock5, with an inhibition of the activation of Rac by Dock5 in the cells 293T of about 95% at a 100 µM concentration. For this test carried out with mouse DHR2 of Dock5, IC₅₀ is 17 µM for E73 (Figure 2).

**Table 1a: Inhibitory effect of the compounds tested on mouse and human Dock5 activity: 048 ED series.**

| | | | | **Inhibition Dock5 (%)** |
|---|---|---|---|---|
| **Structure** | **Code** | **Mol Weight** | **Compounds references** | **Human** |
| 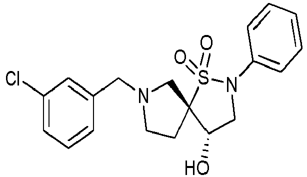 | 048ED0011 | 392,8 | E73 | 70%/ 60% / 55% |
| C₁₉H₂₁ClN₂O₃S | | | | |
| 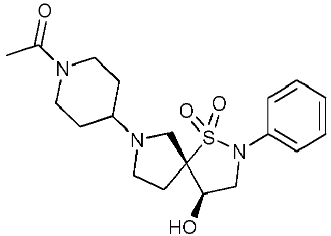 | 048ED0031 | 393,5 | E78 | Negative |
| C₁₉H₂₇N₃O₄S | | | | |
| 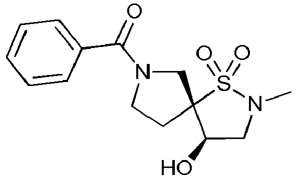 | 048ED0071 | 310,3 | E88 | Negative |
| C₁₄H₁₈N₂O₄S | | | | |

The same approach was used to test the potency of molecules of the 48ED family, on the activity of human Dock5 DHR2 (Table 2).

**Table 2: Inhibitory activity of the compounds of the 48ED family on human Dock5 activity.**

| **Structure** | **Code** | **Mol Weight** | **Compounds references** | **Inhibition** |
|---|---|---|---|---|
| 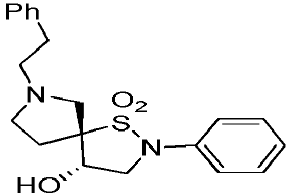 | 048ED0004 | 372,15 | E217 | 25% |
| C₂₀H₂₄N₂O₃S | | | | |
| 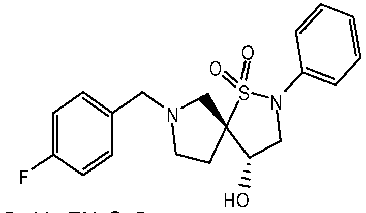 | 048ED0008 | 376,445 | E127 | 40% /50% / 85% / 90% |
| C₁₉H₂₁ FN₂O₃S | | | | |
| 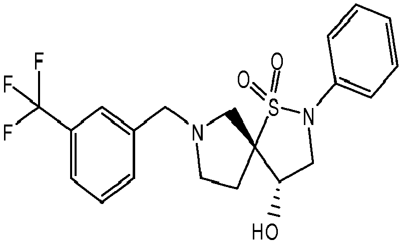 | 048ED0009 | 426,453 | E128 | 90% / 70% at 50µM |
| C₂₀H₂₁ F₃N₂O₃S | | | | |
| 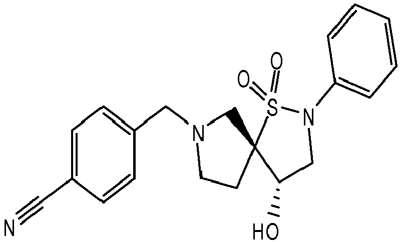 | 048ED0010 | 383,464 | E129 | 70% / 90% / 95% |
| C₂₀H₂₁ N₃O₃S | | | | |
| 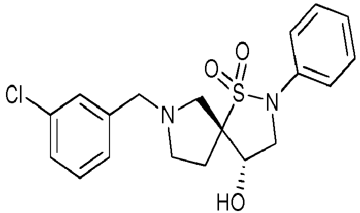 | 048ED0011 | 392,8 | E73 | 55% / 95% / 50% / 90%/95% / 90%/ 95% |
| C₁₉H₂₁ClN₂O₃S | | | | |
| 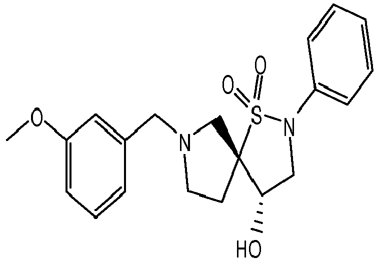 | 048ED0012 | 388,481 | E130 | 60%/ 80% /95% / 90% |
| C₂₆H₂₄N₂O₄S | | | | |
| 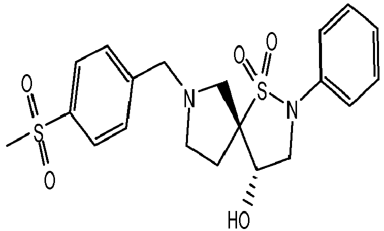 | 048ED0014 | 436,545 | E131 | 50% / 70% /90% |
| C₂₀H₂₄N₂O₅S₂ | | | | |
| 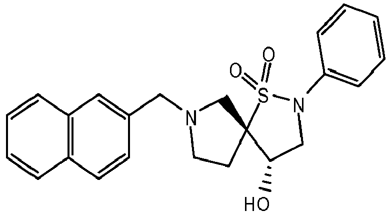 | 048ED0016 | 408,513 | E132 | 70%/ 50% at 10µM/ 60% at 10µM |
| C₂₃H₂₄N₂O₃S | | | | |
| 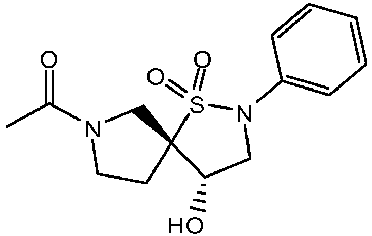 | 048ED0017 | 310,369 | E133 | 5% |
| C₁₄H₁₈N₂O₄S | | | | |
| 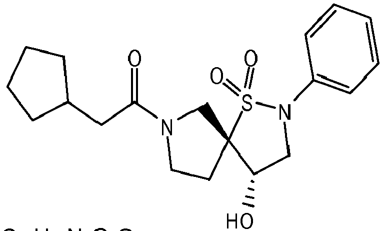 | 048ED0019 | 378,486 | E135 | 70% inhibition, 50% inhibition, 95% inhibition |
| C₁₉H₂₆N₂O₄S | | | | |
| 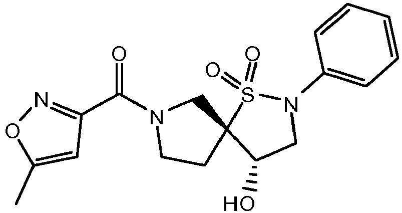 | 048ED0020 | 377,415 | E136 | 30% |
| C₁₇H₁₉N₃O₅S | | | | |
| 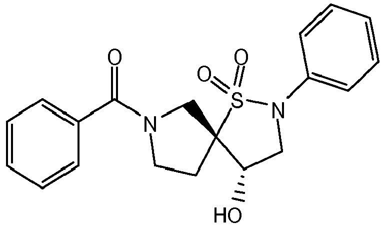 | 048ED0021 | 372,438 | E137 | 70% inhibition, 40% inhibition, 15% inhibition |
| C₁₉H₂₀N₂O₄S | | | | |
| 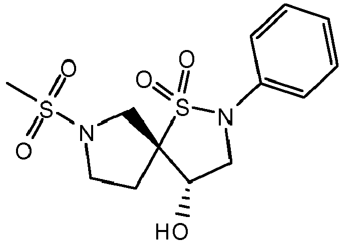 | 048ED0026 | 346,422 | E138 | 30% inhibition |
| C₁₃H₁₈N₂O₅S₂ | | | | |
| 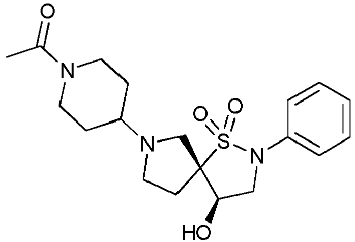 | 048ED0031 | 393,5 | E78 | Negative |
| C₁₉H₂₇N₃O₄S | | | | |
| 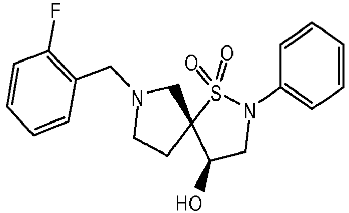 | 048ED0040 | 376,445 | E139 | 60% inhibition, 10% inhibition, |
| C₁₉H₂₁FN₂O₃S | | | | |
| 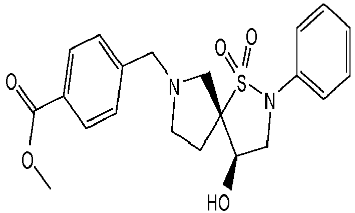 | 048ED0041 | 416,491 | E140 | 50% inhibition, |
| C₂₁ H₂₄N₂O₅S | | | | |
| 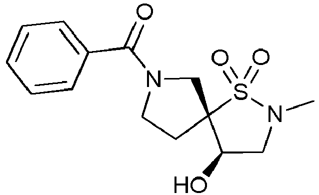 | 048ED0071 | 310,3 | E88 | Negative |
| C₁₄H₁₈N₂O₄S | | | | |
| 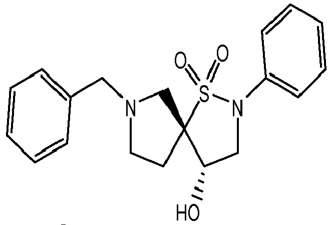 | 048ED0084 | 358,5 | E118 | 85%/ 60% /55% /85% |
| C₁₉H₂₂N₂O₃S | | | | |
| 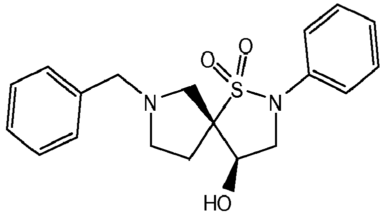 | 048ED0085 | 358,5 | E119 | 25% inhibition |
| C₁₉H₂₂N₂O₃S | | | | |
| 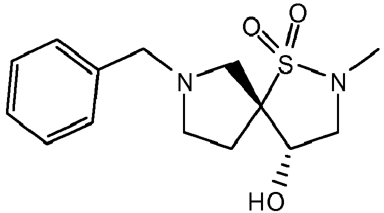 | 048ED0086 | 296,385 | E120 | Negative |
| C₁₄H₂₀N₂O₃S | | | | |
| 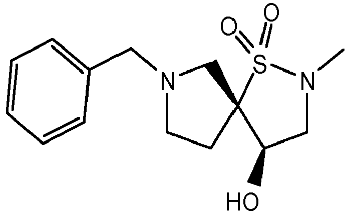 | 048ED0087 | 296,385 | E121 | Negative |
| C₁₄H₂₀N₂O₃S | | | | |
| 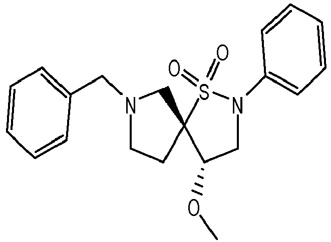 | 048ED0088 | 372,481 | E122 | 30% inhibition |
| C₂₆H₂₄N₂O₃S | | | | |
| 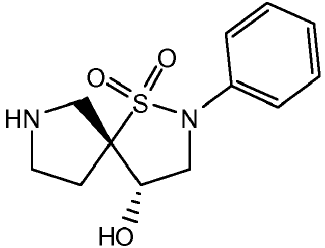 | 048ED0089 | 268,332 | E123 | 10% inhibition |
| C₁₂H₁₆N₂O₃S | | | | |
| 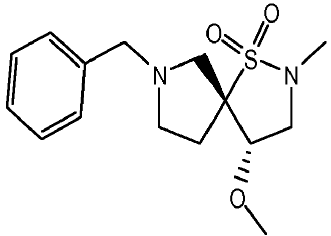 | 048ED0091 | 310,412 | E124 | Negative |
| C₁₅H₂₂N₂O₃S | | | | |
| 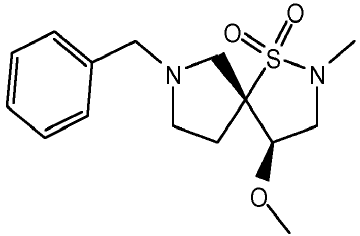 | 048ED0092 | 310,412 | E125 | Negative |
| C₁₅H₂₂N₂O₃S | | | | |
| 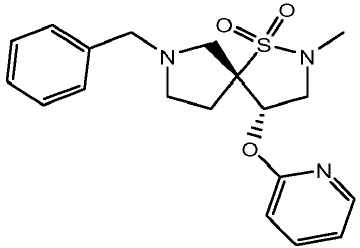 | 048ED0093 | 373,469 | E126 | 10% inhibition |
| C₁₉H₂₃N₃O₃S | | | | |

### EXAMPLE 2 : Structure function analysis of the family 48ED, design of new compounds and inhibitory effect on Dock5: the 268EDL family.

From the structure of the compounds tested in series 048ED, we carried out a structure activity relationship on the 3 residues R1 to R3 and on the core characterizing the family. new compounds were synthesized for this study, identified as 268EDL series (Table 3).

**Table 3: Inhibitory activity of compounds of the 268EDL series on human Dock5 activity.**

| **Structure** | **Code** | **Reference compounds** | **Inhibition** |
|---|---|---|---|
| 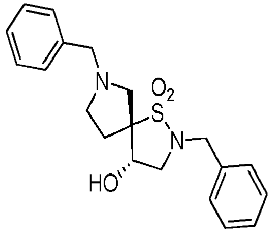 | 268EDL010 | E216 | 40% inhibition, 30% inhibition |
| C₂₆H₂₄N₂O₃S | | | |
| 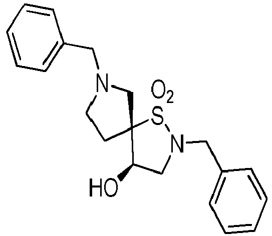 | 268EDL011 | E215 | 85% inhibition, |
| C₂₆H₂₄N₂O₃S | | | |
| 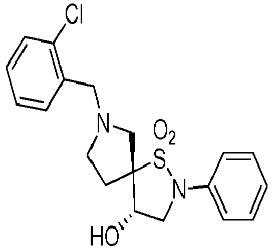 | 268EDL016 | E196 | 95% / 35% / 95% / 40% à 50µM |
| C₁₉H₂₁ClN₂O₃S | | | |
| 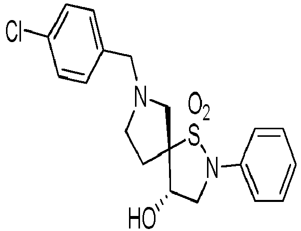 | 268EDL017 | E197 | 55% /90% / 80% à 50µM |
| C₁₉H₂₁ClN₂O₃S | | | |
| 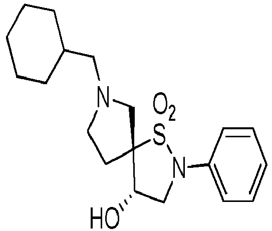 | 268EDL018 | E198 | 95% / 10%/85% |
| C₁₉H₂₈N₂O₃S | | | |
| 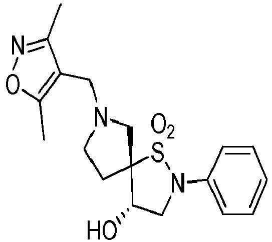 | 268EDL019 | E199 | 40% / 40% |
| C₁₈H₂₃N₃O₄S | | | |
| 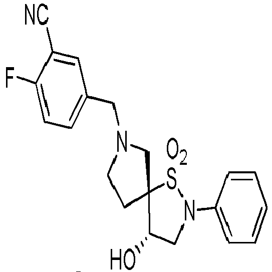 | 268EDL020 | E200 | 75%/ 35% / 95% / 55% at 50µM |
| C₂₆H₂₆FN₃O₃S | | | |
| 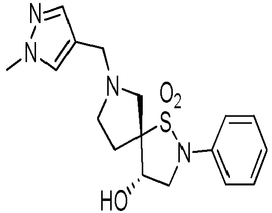 | 268EDL021 | E201 | 60% inhibition, 10% inhibition, 10% inhibition |
| C₁₇H₂₂N₄O₃S | | | |
| 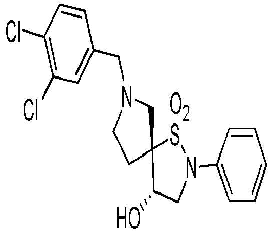 | 268EDL022 | E202 | 95% at 50µM/ 90% at 50µM |
| C₁₉H₂₀Cl₂N₂O₃S | | | |
| 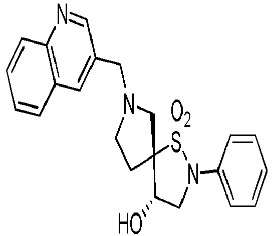 | 268EDL023 | E203 | 95% at 50µM / 90% at 50µM |
| C₂₂H₂₃N₃O₃S | | | |
| 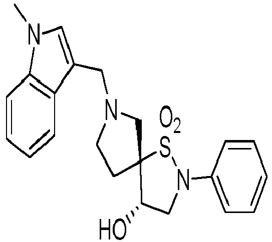 | 268EDL024 | E204 | 100% / 95% / 100% / 60% at 50µM |
| C₂₂H₂₅N₃O₃S | | | |
| 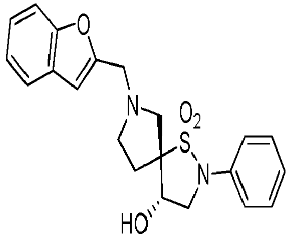 | 268EDL025 | E205 | 100% / 100%/ 70% at 50µM |
| C₂₁ H₂₂N₂O₄S | | | |
| 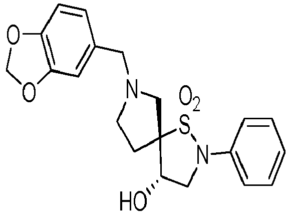 | 268EDL026 | E206 | 50% / 100% / 70% |
| C₂₀H₂₂N₂O₅S | | | |
| 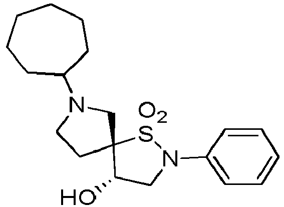 | 268EDL027 | E207 | 20% inhibition |
| C₁₉H₂₈N₂O₃S | | | |
| 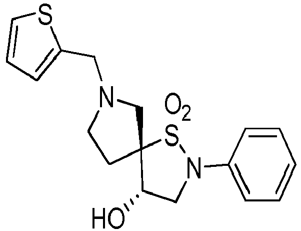 | 268EDL028 | E208 | 35% inhibition |
| C₁₇H₂₀N₂O₃S₂ | | | |
| 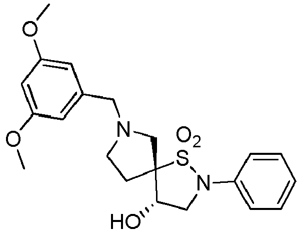 | 268EDL029 | E209 | 35% inhibition |
| C₂₁ H₂₆N₂O₅S | | | |
| 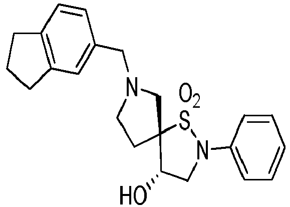 | 268EDL030 | E210 | 85% at 50µM |
| C₂₂H₂₆N₂O₃S | | | |
| 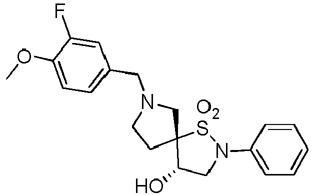 | 268EDL031 | E211 | 90% at 50µM |
| C₂₀H₂₃FN₂O₄S | | | |
| | | | |
| 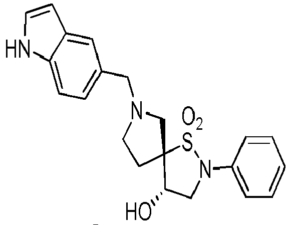 | 268EDL033 | E212 | 98% / 100% |
| C₂₁ H₂₃N₃O₃S | | | |
| 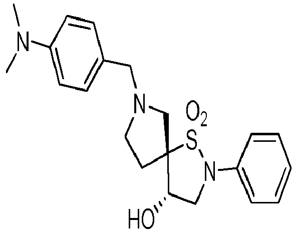 | 268EDL034 | E213 | 90%/ 90% |
| C₂₁ H₂₇N₃O₃S | | | |
| 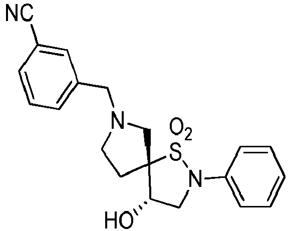 | 268EDL035 | E214 | 90% / 50% |
| C₂₀H₂₁ N₃O₃S | | | |
| 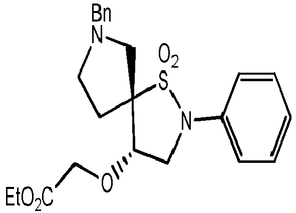 | 268EDL040+045 | E231 | 70% à 50µM |
| C₂₃H₂₈N₂O₅S | | | |
| 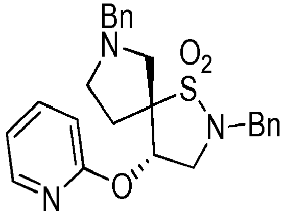 | 268EDL043 | E224 | 20% inhibition |
| C₂₅H₂₇N₃O₃S | | | |
| 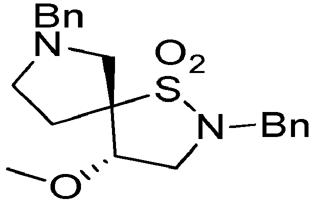 | 268EDL044 | E225 | 35% inhibition |
| C₂₁ H₂₆N₂O₃S | | | |
| 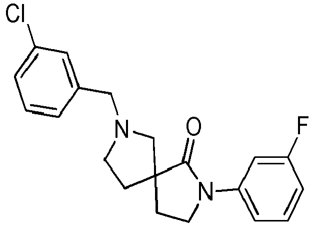 | 268EDL051 | E226 | 55% at 50µM |
| C₂₀H₂₀ClFN₂O | | | |
| 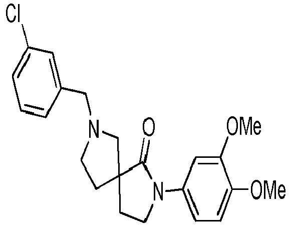 | 268EDL052 | E227 | 90% inhibition, 50% inhibition, 60% inhibition at 100µM |
| C₂₂H₂₅ClN₂O₃ | | | |
| 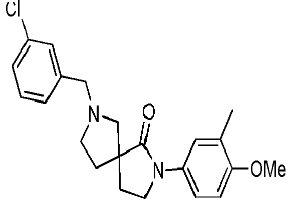 | 268EDL053 | E228 | 65% inhibition at 50µM |
| C₂₂H₂₅ClN₂O₂ | | | |
| 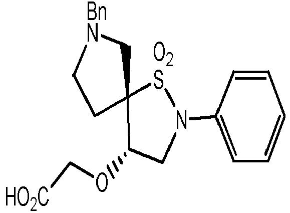 | 268EDL054 | E232 | 40% inhibition, 40% inhibition, 10% inhibition |
| C₂₁ H₂₄N₂O₅S | | | |
| 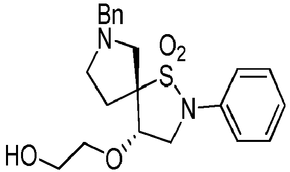 | 268EDL055 | E229 | 65% inhibition at 50µM |
| C₂₁ H₂₆N₂O₄S | | | |
| 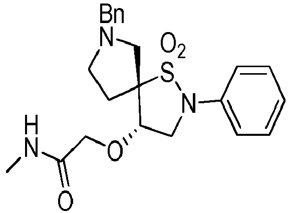 | 268EDL056 | E230 | 80%/ 45% / 30% / 70% |
| C₂₂H₂₇N₃O₄S | | | |
| 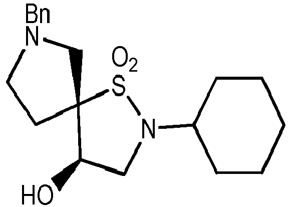 | 268EDL059+061 | E234 | 85% /55% / 40% / 60% |
| C₁₉H₂₈N₂O₃S | | | |
| 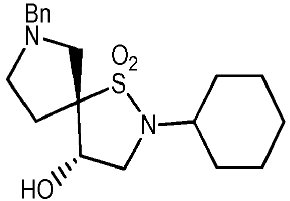 | 268EDL060 | E233 | 70% /60% / 60% |
| C₁₉H₂₈N₂O₃S | | | |
| 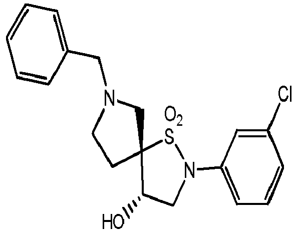 | 268EDL070 | E235 | 80%/ 85% |
| C₁₉H₂₁ClN₂O₃S | | | |
| 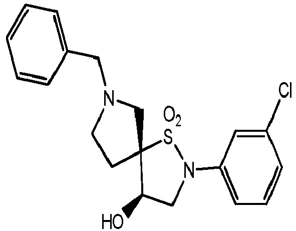 | 268EDL071 | E236 | 70% inhibition, 20% inhibition à 50µM |
| C₁₉H₂₁ClN₂O₃S | | | |

For the 11 most active products among the 2 families 048ED and 268EDL, IC50 were determined on the human Dock5 (Table 4) according to the method described in Figure 1, using a range of doses as in Figure 2.

**Table 4: IC50 (µM) for the 11 compounds most active on human Dock5**

| **1 hour treatment** | **IC50 (µM)** |
|---|---|
| **E73** | 36 |
| **E196** | 35 |
| **E197** | 36 |
| **E202** | 29 |
| **E203** | 22 |
| **E204** | 33 |
| **E205** | 43 |
| **E210** | 32 |
| **E211** | 44 |
| **E226** | 20 |
| **E228** | 8 |

The IC50 (µM) of these most active products, tested in dose response, are comprised between 8 and 44 µM.

### EXAMPLE 3: Effect of the molecules on the activation of Rac1 by Dock5, in vitro test using purified proteins.

The effect of the molecules has been tested on the nucleotide exchange reaction by the GTPase Rac1 catalyzed by the DHR2 exchange domain of Dock5 or by the Dbl exchange domain of TRIO, using purified recombinant proteins produced in E. coli bacteria (Vives, Cres et al., 2015).

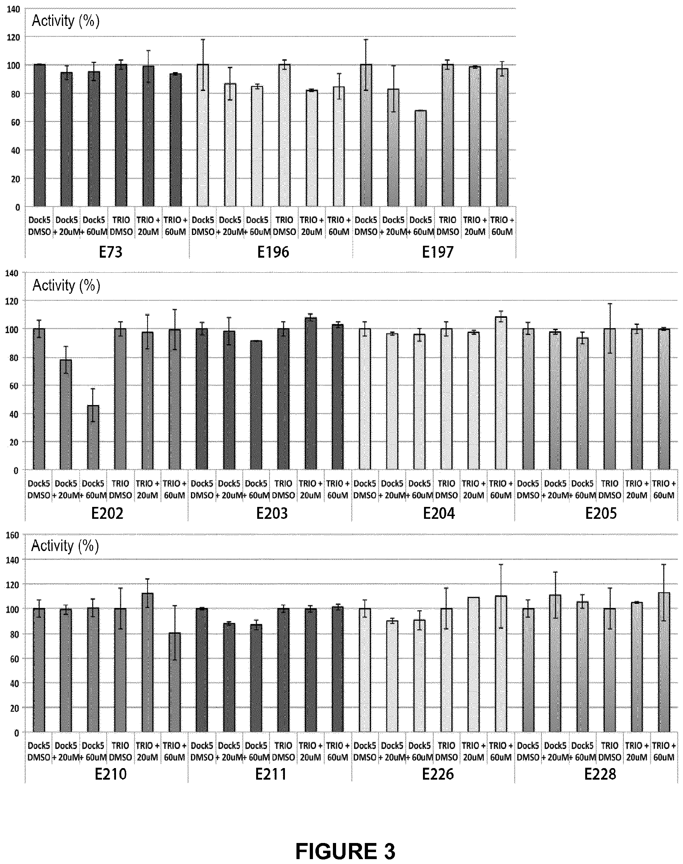

The activity of the inhibitors is measured by fluorescence spectroscopy on 96-well plates with a FlexStation 3 fluorimeter, in a reaction volume of 150 µL, without stirring, at a temperature of 26 ° C. Each plate contains 4 inhibitors at two concentrations (20 and 60 µM), 2 GEFs (DOCK5 and TRIO) and includes controls without inhibitor (1% final DMSO). The inhibitors are diluted to DMSO at an intermediate concentration of 2 or 6 mM, ie 1% final DMSO. Rac1-mantGDP (2 µM), DOCK5 or TRIO (0,2 µM) and the inhibitors are mixed immediately before the beginning of measurements. The exchange reaction is triggered by series of 8 wells in parallel by addition of 100 µM of GTP with mixing by pipetting (Flex mode). The kinetics is followed during 800 seconds. All the measurements are made in duplicate on the same plate. The total playing time of a plate is about 80 minutes. The kinetics of exchange are analyzed with a mono-exponential model F = F0 + ΔFe - kt with the KaleidaGraph ™ software, where F0 is the initial fluorescence and k (or kobs) is the exchange rate constant. The residual activity for each concentration of inhibitor is determined by the ratio: kobs with inhibitor / kobs without inhibitor and expressed as a percentage (Figure 3).The molecules E197 and E202 were shown to be active in this test: they inhibit Dock 5 and not TRIO.

### EXAMPLE 4: Effect of the molecules on the bone resorption activity of human osteoclasts in culture.

A model of in vitro osteoclast differenciation from human peripheral blood monocytic cells was used. Tested compounds were E197 and E202. They were compared to Alendronate, a bisphosphonate of reference.

Monocytic cells are purified from the peripheral blood of healthy donors, obtained with the agreement of l'Etablissement Français du Sang (the French Blood Establishment). The purification protocol comprises 2 steps:
1/ Mononuclear cell isolation from peripheral blood by Ficoll,
2/ Monocytic cell selection (cells CD14+) by magnetic sorting (MACS®, MiltenyiBiotec).

The monocytes are differentiated into osteoclasts by adding αMEM 10% SVF medium supplemented with M-CSF (25 ng / mL) and RANKL (100 ng / mL) (differentiation medium). The differentiation time is on average 6 days.

At day 4, cells have been detached with accutase and then reseeded.
- On a surface coated with synthetic mineral matrix (96- wells plate) for the evaluation of resorption and,
- On a plastic surface (96- wells plate) to quantify osteoclast numbers.

After sedimentation of the cells, the medium was renewed with differentiation medium with or without the test compounds and the cells were grown for 48 hours.

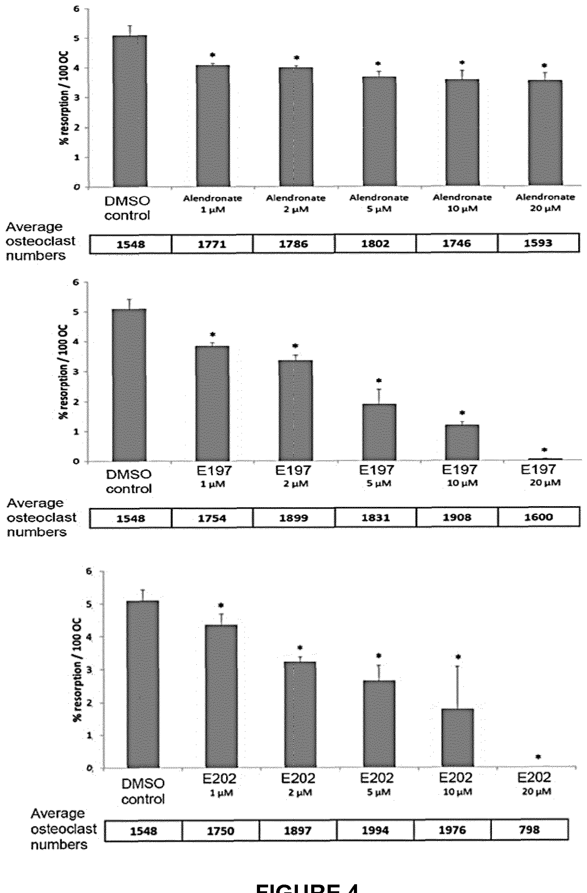

The two molecules have been found more efficient than the Alendronate to inhibit the human osteoclasts specific activity (Figure 4).

### EXAMPLE 5: Effect of the molecule E197 on bone dynamics parameters in ovariectomized mice.

The ovariectomized mouse was used as an *in vivo* model of pathological bone loss. Tested compound was E197; it was compared to Zoledronate, a bisphosphonate of reference.

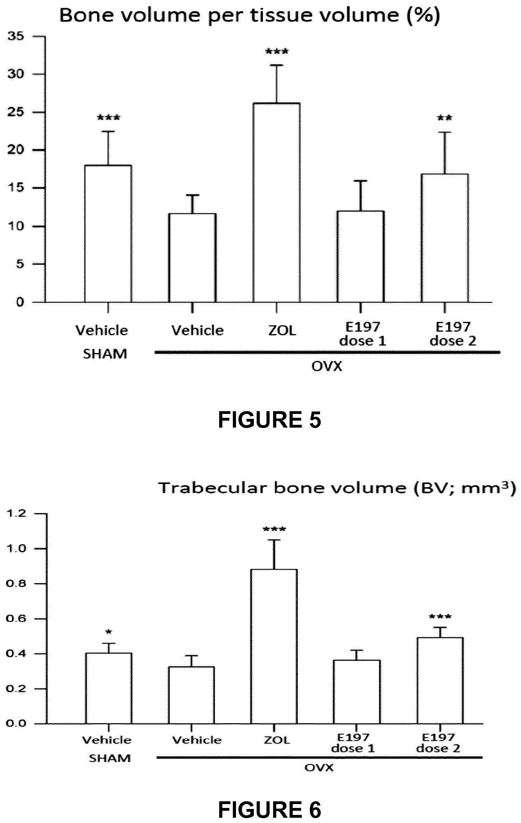

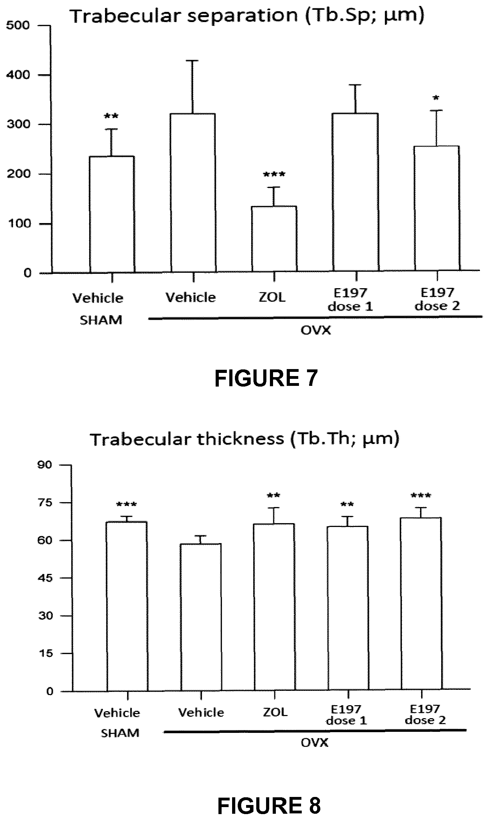

Each group contained ten female C57BL/6J mice that were fourteen weeks of age at the beginning of in-life phase of the study. At the beginning of the in-life phase (at study day 0), mice were weighed and their surgical ovariectomy (OVX) and control (SHAM) operations were performed under analgesia and anaesthesia. E197 was injected intra peritoneally (i.p.) to OVX animals, 20 mg/kg once (q.d., dose 1) or twice (b.i.d., dose 2) daily for 25 days, in a vehicle solution containing 0.485% carboxymethylcellulose (CMC) and 3% Tween 80. Control mice received the same volume of vehicle. The effects of treatment with reference compound zoledronate (100 µg/kg, subcutaneously, twice a week.) were studied by comparing OVX mice treated with zoledronate and vehicle with OVX control mice treated with vehicle. At study days 21 and 26, bone was labelled with subcutaneous injections of oxytetracycline and calcein green, respectively, in order to enable the analysis of optional dynamic parameters in bone histomorphometry. Blood for serum samples were harvested from saphenous vein at days -1, 13 and 27 of the study for bone dynamics marker dosage. The amount, microarchitecture, cellular characteristics and metabolic activity of metaphyseal trabecular bone and the amount and metabolic activity of diaphyseal cortical bone were analyzed by bone histomorphometry in femora, and the volume, cross-sectional dimensions and microarchitecture of metaphyseal trabecular bone and the volume and cross-sectional dimensions of diaphyseal cortical bone were analyzed by high-resolution µCT in tibiae using terminal bone samples harvested at the end of the in-life phase (at study day 28).
- Treatment with test compound E197 at 20 mg/kg, i.p., b.i.d. prevented the OVX-induced reduction in the amount of trabecular bone in long bone metaphysis. This finding was observed by bone histomorphometry and high-resolution µCT as increased trabecular bone volume fraction (BV/TV) in femoral and tibial metaphyses, respectively, at the end of the in-life phase in OVX mice treated with the compound compared with OVX control mice treated with vehicle (Figures 5 & 6).
- Treatment with test compound E197 at 20 mg/kg, i.p., b.i.d. increased the number of individual trabeculae in tibial metaphysis. This finding was observed by high-resolution µCT as increased trabecular number (Tb.N) in tibial metaphysis at the end of the in-life phase in OVX mice treated with the compound compared with OVX control mice treated with vehicle. The observation was identified also by bone histomorphometry as decreased trabecular separation (Tb.Sp) demonstrating decreased distance between two individual trabeculae in OVX mice treated with the compound (Figures 7 & 8).

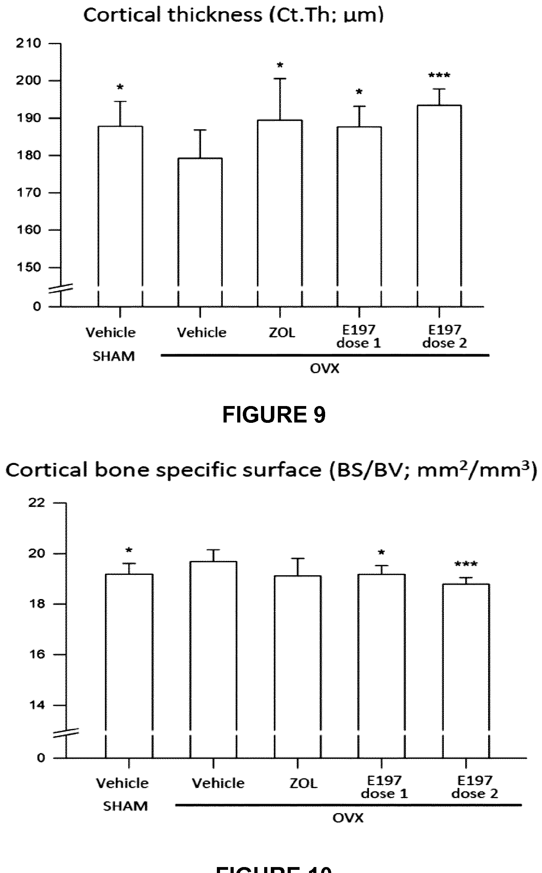

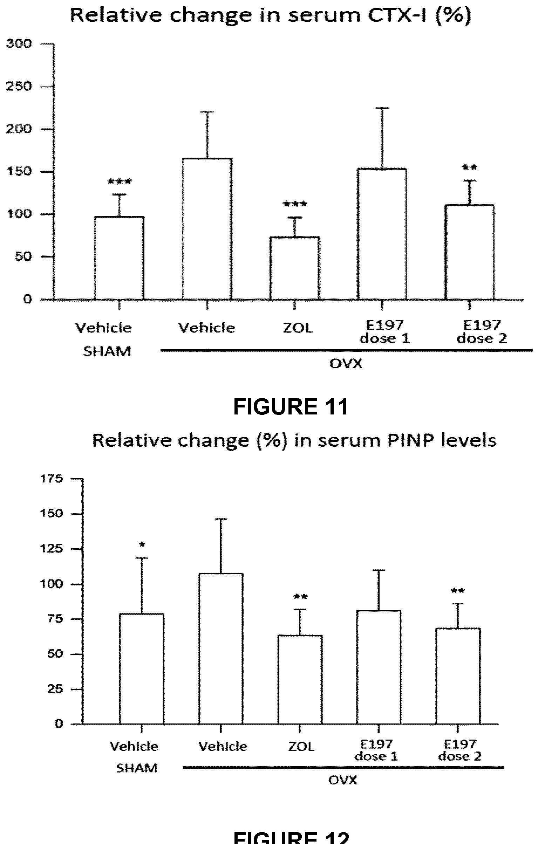

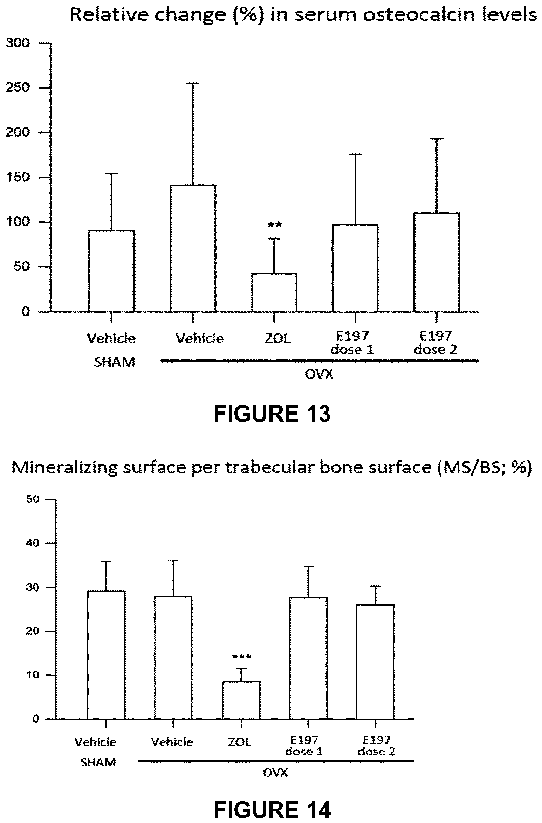

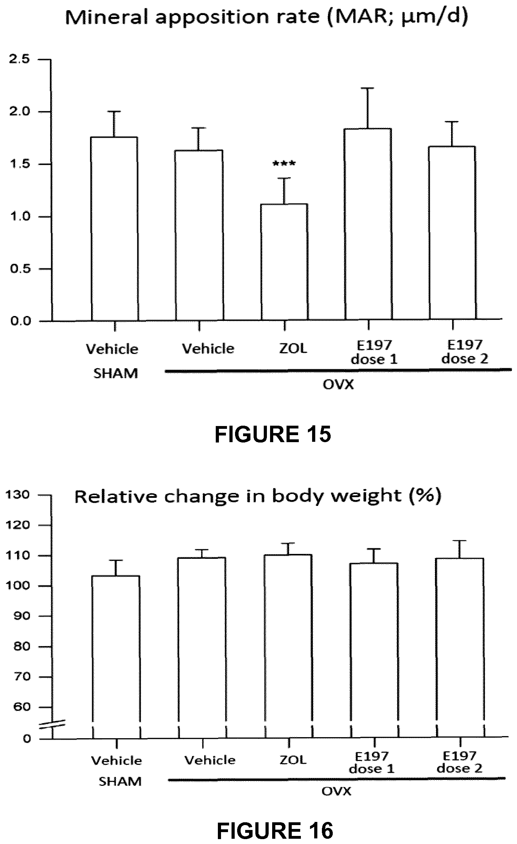

- Treatment with test compound E197 at 20 mg/kg, i.p., q.d. and b.i.d. prevented the OVX-induced reduction in the amount of cortical bone in tibial diaphysis. This finding was observed by high-resolution µCT as increased cortical thickness (Ct.Th) and decreased cortical bone specific surface (BS/BV) in tibial diaphysis at the end of the in-life phase in OVX mice treated with the compound compared with OVX control mice treated with vehicle. (Figures 9 & 10).
- At the whole body level, treatment with test compound E197 at 20 mg/kg, i.p., b.i.d. prevented the OVX-induced increase in serum levels of bone resorption biomarker CTX-I during the first two weeks of in-life phase of the study. This finding was observed by the measurements of serum CTX-I levels as decreased relative change in serum CTX-I levels during the first two weeks of the in-life in OVX mice treated with the compound compared with OVX control mice treated with vehicle (Figure 11).
- At the whole body level, treatment with test compound E197 at 20 mg/kg, i.p., b.i.d. prevented the OVX-induced increase in serum levels of bone formation biomarker PINP during the first two weeks of the in-life phase and decreased serum PINP levels during the entire in-life phase. This finding was observed by the measurements of serum PINP levels as decreased relative change in serum PINP levels during the first two weeks of the in-life phase and the entire in-life phase in OVX mice treated with the compound compared with OVX control mice treated with vehicle (Figure 12).
- At the whole body level, treatment with test compound E197 at 20 mg/kg, i.p., q.d. and b.i.d. did not decreased serum levels of bone turnover biomarker osteoclacin during the entire in-life phase, contrarily to reference compound Zoledronate. This finding was observed by the measurements of serum osteoclacin levels as decreased relative change in serum osteoclacin levels during the in-life phase in OVX mice treated with the compound and vehicle compared with OVX controle mice treated with vehicle (Figure 13).
- Treatment with E197 at 20 mg/kg, i.p., q.d. and b.i.d did not decreased the surface and ratio of trabecular bone mineralization contributing to the decreased trabecular bone formation in femoral metaphysis, contrarily to reference compound Zoledronate. This finding was observed by bone histomorphometry as decreased mineralizing surface per bone surface (MS/BS) and decreased mineral apposition rate (MAR) in trabecular bone in femoral metaphysis at the end of the in-life phase in OVX mice treated with the compound and vehicle compared with OVX control mice treated with vehicle. (Figures 14 & 15).

These results demonstrated that treatment with test compound E197 at 20 mg/kg, i.p., b.i.d. prevented the OVX-induced reduction in the amount of trabecular bone in long bone metaphysis in young adult OVX mice. In addition, the results demonstrated that treatment with test compound E197 at 20 mg/kg, i.p., q.d. and b.i.d. prevented the OVX-induced reduction in the amount of cortical bone. In addition, the results demonstrated that treatment with test compound E197 at 20 mg/kg, i.p., q.d. and b.i.d. did not affect trabecular bone formation, contrarily to reference compound Zoledronate. At the whole body level, this test compound E197 treatment prevented the OVX-induced increase in bone resorption and formation. These findings indicated a significant anti-catabolic activity for test compound E197 at 20 mg/kg, i.p., b.i.d. both in metaphyseal trabecular bone and diaphyseal cortical bone in young adult OVX mice, this without affecting the bone formation rate, contrarily to reference compound Zoledronate. Treatment with test compound E197 did not affect body weight (Figure 16).

### EXAMPLE 6 : Synthesis of some candidates compounds of the invention

### 6.1 General synthesis process

The compounds of formula (I) may be obtained by the following protocol:

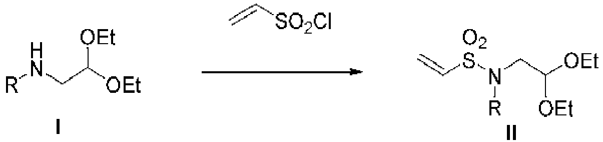

2-chloroethane-1-sulfonyl chloride (1.3 eq) was added to a cooled (0°C) solution of **I** (1 eq) and triethylamine (3 eq) in DCE (0.1 M). The reaction mixture was stirred at room temperature overnight. The suspension was filtered and the organic layer was washed with saturated aqueous sodium bicarbonate, with aqueous HCl 1N, with brine, dried over sodium sulphate, filtered and the filtrate was concentrated under vacuum to afford title compound **II.**

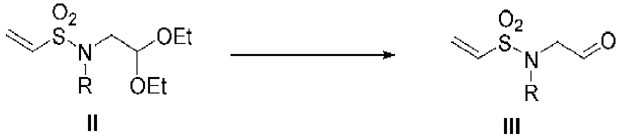

A solution of **II** (1 eq) was stirred for three days in a mixture of acetic acid / THF / 10% aqueous HCI (1 / 1 / 2; 0.1 M). The solution was poured onto EA and the two phases were separated. The organic layer was washed with water, with brine, dried over sodium sulphate, filtered and the filtrate was concentrated under vacuum. Purification of the residue by flash chromatography on silica gel afforded the title compound **III.**

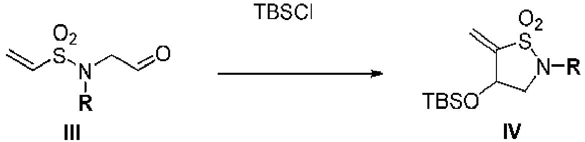

A solution of **III** (1 eq) and DABCO (0.1 eq) in DCM (0.2 M) was stirred at room temperature. After two hours, imidazole (1.4 eg) followed by tert-butyldimethylsilyl chloride (1.4 eq) were added. After two hours of stirring, additional imidazole (0.1 eq) and tert-butyldimethylsilyl chloride (0.1 M) were added. Once the conversion complete, the suspension was filtered off and the filtrate was concentrated under vacuum. Purification of the residue by flash chromatography on silica gel afforded the title compound **IV.**

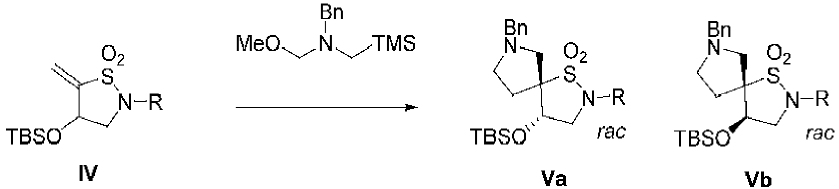

Trifluoroacetic acid (0.1 eq) was added to a solution of **IV** (1.0 eq) and N-(methoxymethyl)-*N*-(trimethylsilylmethyl)benzylamine (1.5 eq) in DCE (0.1 M). The resulting solution was stirred at 45°C overnight. After cooling to room temperature, the organic layer was washed with saturated aqueous sodium bicarbonate, with brine, dried over sodium sulphate, filtered and the filtrate was concentrated under vacuum. Purification of the residue by flash chromatography on silica gel afforded the title compound **Va** and **Vb.**

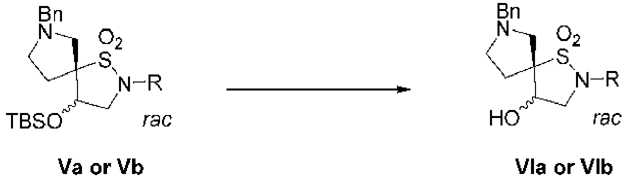

A solution of tetrabutylammonium fluoride (1M in THF, 1.2 eq) was added to a solution of **V** (1.0 eq) in THF (0.1 N). After complete consumption of starting material, the medium was concentrated under reduced pressure. Purification of the residue by flash chromatography on silica gel afforded the title compound **VI.**

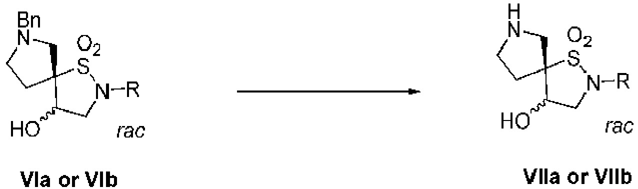

A solution of **VI** (1.0 eq) in a mixture of EtOH and acetic acid (9/1, 0.05 M) was hydrogenated using Palladium (Pd/C 10%) as catalyst in a ThalesNano, H-cube® system (40 bars, 40°C, H2, flow rate of 0.8 mL/min). Concentration of the resulting solution and purification of the residue by filtration on silica gel afforded the title compound **VII.**

### Typical procedure for reductive amination

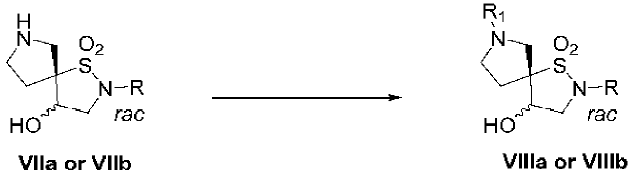

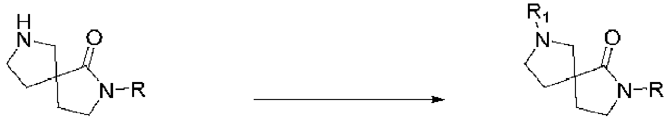

A suspension of **VII** (1 eq) and the aldehyde/ketone (1.2 eq) in THF (0.15 M) was stirred at room temperature for 30 minutes. Sodium triacetoxyborohydride (2.2 eq) was then added and the resulting mixture was stirred at room temperature overnight. The medium was treated with water, the suspension was filtered and the filtrate was concentrated under reduced pressure. Purification of the residue by preparative RP-HPLC (Puriflash) afforded desired compound **VIII.**

### Typical procedure for acylation/sulfonylation

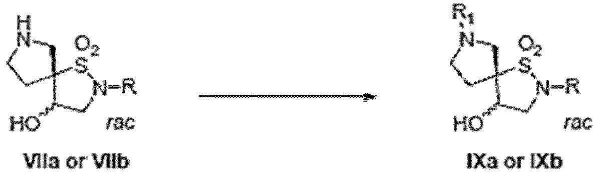

suspension of **VII** (1 eq), triethylamine (1.2 eq) and the acyl/sulfonyl chloride (1.2 eq) in THF (0.15 M) was stirred at room temperature overnight. The suspension was filtered and the filtrate was concentrated under reduced pressure. Purification of the residue by preparative RP-HPLC (Puriflash) afforded desired compound **IX.**

### Typical procedure for O-alklyation

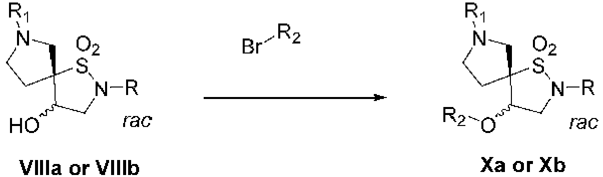

To a cooled (0°C) solution of **VIII** (1 eq) in THF (0.1 M) was added NaH (1.5 eq). After 15 minutes of stirring, the alkyl bromide (1.5 eq) was added and the medium was allowed to warm to room temperature. Once the reaction was complete, the medium was quenched by addition of acetic acid. The mixture was poured in EA. The organic layer was washed with an aqueous sat solution of NaHCO3, with brine, dried over sodium sulfate and concentrated under reduced pressure. Purification of the residue by column chromatography on silica gel afforded desired compound **X.**
¹H NMR spectra were recorded on a Bruker AVANCE 500 NMR Spectrometer equipped with a Bruker 5 mm PABBO BB-1H/D Z-GRD at 500MHz for proton NMR. Chemical shifts are reported in ppm (δ). Data are reported as follows: chemical shifts (δ), multiplicity (b = broad, s = singlet, d= doublet, t = triplet, q = quartet, quint = quintuplet, m = multiplet), coupling constants (J) in Hz, integration.

Compounds were purified by Semi preparative LCMS. MS instrument type: Waters QDA (ESI source); HPLC instrument type: Waters 2525 with « Make up » and « At column » pumps 515; Photodiode Array Detector Waters 2996; column: Waters XSelect CSH C18 OBD, 30x50mm, 5µm; mobile phase A: 10 mM ammonia in water, mobile phase B: acetonitrile; flow rate: 60 ml/min; injection loop volume: 2 mL.

UPLC spectra were acquired on a Waters 3100; UHPLC instrument type: Waters Acquity HClass; UV PDA eλ Detector; column: Waters XSelect C18 CSH, 2.1x50mm, 2.5µm; mobile phase A: 10 mM ammonia in water, mobile phase B: acetonitrile; gradient: 0.0 min 95% A → 0.5 min 95% A → 3.15 min 2% A → 3.42 min 2% A → 3.67 min 95% A → 4.0 min 95% A; flow rate: 1.0 ml/min; detection Thermo Corona Ultra RS.

### 6.2 Detailed synthesis of compounds E196, E197, E202, E203, E204, E205, E210, E211

### N-2,2-diethoxyethyl)-N-phenylethenesulfonamide

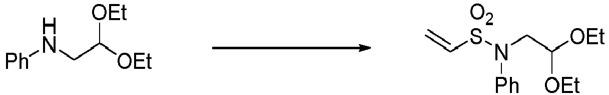

2-chloroethane-1-sulfonyl chloride (13.07 mL, 124.2 mmol) was added to a cooled (0°C) solution of *N*-(2,2-diethoxyethyl)aniline (20.0 g, 95.5 mmol) and triethylamine (39.96 mL, 286.7 mmol) in DCE (1 L). The reaction mixture was stirred at room temperature overnight. The suspension was filtered and the organic layer was washed with saturated aqueous sodium bicarbonate, with aqueous HCI 1N, with brine, dried over sodium sulphate, filtered and the filtrate was concentrated under vacuum to afford title compound (28.4 g, 99%, brown oil). ¹H NMR (500 MHz, CDCl₃): δ 7.41-7.29 (m, 5H), 6.58 (dd, *J* = 16.6, 9.9 Hz, 1H), 6.16 (d, *J* = 16.6 Hz, 1H), 5.94 (d, *J* = 9.9 Hz, 1H), 4.61 (t, *J* = 5.5 Hz, 1H), 3.69 (d, *J* = 5.5 Hz, 2H), 3.63 (m, 2H), 3.49 (m, 2H), 1.14 (t, *J* = 7.0 Hz, 6H).

### N-(2-oxoethyl)-N-phenylethenesulfonamide

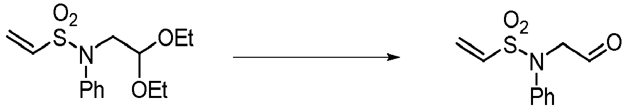

A solution of *N*-(2,2-diethoxyethyl)-*N*-phenylethenesulfonamide (28.4 g, 94.9 mmol) was stirred for three days in a mixture of acetic acid (200 mL), THF (200 mL) and 10% aqueous HCI (400 mL). The solution was poured onto EA and the two phases were separated. The organic layer was washed with water, with brine, dried over sodium sulphate, filtered and the filtrate was concentrated under vacuum. Purification of the residue by flash chromatography on silica gel (cHx-EA 1/1) afforded the title compound (15.30 g, 71%, orange oil). ¹H NMR (500 MHz, CDCl₃): δ 9.69 (s, 1H), 7.40-7.30 (m, 5H), 6.66 (dd, *J* = 16.6, 9.9 Hz, 1H), 6.16 (d, *J* = 16.6 Hz, 1H), 5.99 (d, *J* = 9.9 Hz, 1H), 4.41 (s, 2H)

### 4-((tert-butyldimethylsilyl)oxy)-5-methylene-2-phenylisothiazolidine 1,1-dioxide

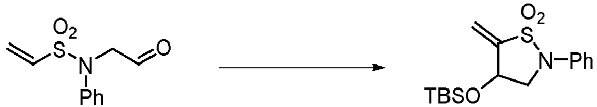

A solution of *N*-(2-oxoethyl)-*N*-phenylethenesulfonamide (15.28 g, 67.8 mmol) and DABCO (0.76 g, 6.8 mmol) in DCM (310 mL) was stirred for two hours at room temperature. Then imidazole (6.47 g, 95.0 mmol) followed by tert-butyldimethylsilyl chloride (14.31 g, 95.0 mmol) were added. After two hours of stirring, additional imidazole (0.46 g, 6.8 mmol) and tert-butyldimethylsilyl chloride (1.02 g, 6.8 mmol) were added. Once the conversion complete, the suspension was filtered off and the filtrate was concentrated under vacuum. Purification of the residue by flash chromatography on silica gel (cHx-EA) afforded the title compound (15.2 g, 66%, orange oil). ¹H NMR (500 MHz, CDCl₃): δ 7.38 (m, 2H), 7.34-7.30 (m, 2H), 7.23-7.18 (m, 1H), 6.23 (m, 1H), 5.87 (dd, *J* = 2.5, 1.7 Hz, 1H), 5.11 (tt, *J* = 7.3, 2.5 Hz, 1H), 3.86 (dd, *J* = 8.4, 7.2 Hz, 1H), 3.53 (dd, *J* = 8.4, 7.5 Hz, 1H), 0.96 (s, 9H), 0.19 (s, 3H), 0.18 (s, 3H).

### Rac-(4S,5R)-7-benzyl-4-((tert-butyldimethylsilyl)oxy)-2-phenyl-1-thia-2,7-diazaspiro[4.4]nonane 1,1-dioxide

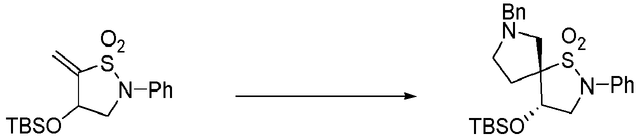

Trifluoroacetic acid (333 µL, 4.49 mmol) was added to a solution of 4-((tert-butyldimethylsilyl)oxy)-5-methylene-2-phenylisothiazolidine 1,1-dioxide (15.25 g, 44.92 mmol) and *N*-(methoxymethyl)-*N*-(trimethylsilylmethyl)benzylamine (17.24 mL, 67.37 mmol). The resulting solution was stirred at 45°C overnight. After cooling to room temperature, the organic layer was washed with saturated aqueous sodium bicarbonate, with brine, dried over sodium sulphate, filtered and the filtrate was concentrated under vacuum. Purification of the residue by flash chromatography on silica gel (cHx-EA 9/1 to 75/25) afforded the title compound (8.17 g, 38%, yellow oil) and its diastereoisomer (8.10 g, 38%, yellow oil). ¹H NMR (500 MHz, CDCl₃): δ 7.38-7.28 (m, 6H), 7.26-7.21 (m, 3H), 7.12 (tt, *J* = 7.3, 1.1 Hz, 1H), 4.55 (dd, *J* = 7.8, 6.7 Hz, 1H), 3.70-3.64 (m, 3H), 3.43-3.38 (m, 2H), 2.88 (td, *J* = 8.1, 5.6 Hz, 1H), 2.68 (d, *J* = 11.0 Hz, 1H), 2.66-2.59 (m, 1H), 2.52-2.37 (m, 2H), 0.93 (s, 9H), 0.12 (s, 3H), 0.11 (s, 3H).

### Rac-(4S,5R)-7-benzyl-4-hydroxy-2-phenyl-1-thia-2,7-diazaspiro[4.4]nonane 1,1-dioxide

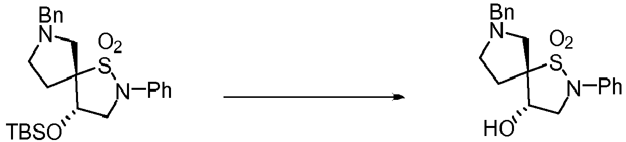

A solution of tetrabutylammonium fluoride (1M in THF, 20.74 mmol, 20.74 mL) was added to a solution of Rac-(4S,5R)-7-benzyl-4-((tert-butyldimethylsilyl)oxy)-2-phenyl-1-thia-2,7-diazaspiro[4.4]nonane 1,1-dioxide (8.17 g, 17.28 mmol) in THF (170 mL). After complete consumption of starting material, the medium was concentrated under reduced pressure. Purification of the residue by flash chromatography on silica gel (cHx/EA: 1/0 to 0/1) afforded the title compound (4.32 g, 70%, orange solid). ¹H NMR (500 MHz, CDCl₃): δ 7.38-7.26 (m, 7H), 7.25-7.20 (m, 2H), 7.14 (tt, *J* = 7.4, 1.1 Hz, 1H), 4.56 (dd, *J* = 6.3, 5.2 Hz, 1H), 3.87 (dd, *J* = 9.3, 6.3 Hz, 1H), 3.71 (d, *J* = 12.9 Hz, 1H), 3.64 (d, *J* = 12.9 Hz, 1H), 3.56 (dd, *J* = 9.3, 5.2 Hz, 1H), 3.07 (d, *J* = 10.0 Hz, 1H), 3.00 (td, *J* = 8.5, 3.8 Hz, 1H), 2.90 (d, *J* = 10.0 Hz, 1H), 2.65 (m, 1H), 2.53 (m, 1H), 2.44 (ddd, *J* = 13.9, 8.8, 3.8 Hz, 1H).

### Rac-(4S,5R)-4-hydroxy-2-phenyl-1-thia-2,7-diazaspiro[4.4]nonane 1,1-dioxide

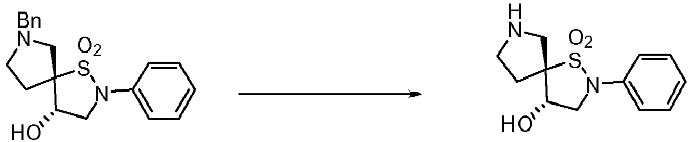

A solution of *Rac*-(4S,5R)-7-benzyl-4-hydroxy-2-phenyl-1-thia-2,7-diazaspiro[4.4]nonane 1,1-dioxide (1.80 g, 5.02 mmmol) in a mixture of EtOH (90 mL) and acetic acid (10 mL) was hydrogenated using Palladium (Pd/C 10%) as catalyst in a ThalesNano, H-cube® system (40 bars, 40°C, H₂, flow rate of 0.8 mL/min). Concentration of the resulting solution and purification of the residue by filtration on silica gel (CH₃CN-aqNH₃ 100% to 95/5) afforded the title compound as a white solid (0.93 g, 69%). ¹H NMR (500 MHz, DMSO-*d*₆): δ 7.41-7.33 (m, 2H), 7.25-7.16 (m, 2H), 7.10 (tt, *J* = 7.4, 1.1 Hz, 1H), 6.09 (d, *J* = 4.9 Hz, 1H), 4.39 (td, *J* = 6.1, 4.9 Hz, 1H), 3.84 (dd, *J* = 9.1, 6.1 Hz, 1H), 3.44 (dd, *J* = 9.1, 6.1 Hz, 1H), 3.38 (d, *J* = 12.6 Hz, 1H), 2.98 (d, *J* = 12.6 Hz, 1H), 2.93 (ddd, *J* = 10.9, 7.7, 5.8 Hz, 1H), 2.85 (ddd, *J* = 10.9, 7.5, 6.3 Hz, 1H), 2.29 (ddd, *J* = 13.4, 7.5, 5.8 Hz, 1H), 2.06 (ddd, *J* = 13.7, 7.7, 6.3 Hz, 1H).

### Typical procedure for reductive amination

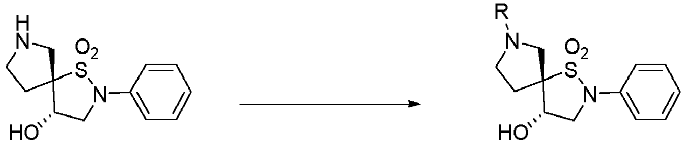

A suspension of Rac-(4S,5R)-4-hydroxy-2-phenyl-1-thia-2,7-diazaspiro[4.4]nonane 1,1-dioxide (0.175 mmol) and the aldehyde/ketone (0.21 mmol, 1.2 eq) in THF (1 mL) was stirred at room temperature for 30 minutes. Sodium triacetoxyborohydride (0.385 mmol, 2.2 eq) was then added and the resulting mixture was stirred at room temperature overnight. The medium was treated with water (100 µL), the suspension was filtered and the filtrate was concentrated under reduced pressure. Purification of the residue by preparative RP-HPLC (Puriflash) afforded desired compound.

The following compounds are obtained by synthesis process described above :

### rac-(4S,5R)-7-(2-chlorobenzyl)-4-hydroxy-2-phenyl-1-thia-2,7-diazaspiro[4.4]nonane 1,1-dioxide (268EDL016 - E196)

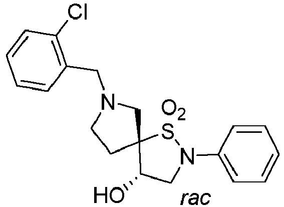

**UPLC-MS (m/z): 393.1-395.2 [M+H]⁺**. ¹H NMR (500 MHz, DMSO-*d*₆): δ 7.54 (dd, *J* = 7.6, 1.8 Hz, 1H), 7.43 (dd, *J* = 7.9, 1.4 Hz, 1H), 7.38-7.33 (m, 3H), 7.29 (td, *J* = 7.6, 1.9 Hz, 1H), 7.22-7.20 (m, 2H), 7.11 (tt, *J* = 7.4, 1.1 Hz, 1H), 6.25 (s, 1H), 4.39 (t, *J* = 6.7 Hz, 1H), 3.81 (dd, *J* = 9.1, 6.3 Hz, 1H), 3.77 (d, *J* = 14.3 Hz, 1H), 3.72 (d, *J* = 14.3 Hz, 1H), 3.44 (dd, *J* = 9.0, 7.0 Hz, 1H), 3.34 (d, *J* = 10.7 Hz, 1H), 2.92 (td, *J* = 8.2, 4.2 Hz, 1H), 2.71 (d, *J* = 10.8 Hz, 1H), 2.54 (q, *J* = 7.9 Hz, 1H), 2.41 (ddd, *J* = 13.6, 7.7, 4.2 Hz, 1H), 2.23 (dt, *J* = 13.6, 7.7 Hz, 1H).

### rac-(4S,5R)-7-(4-chlorobenzyl)-4-hydroxy-2-phenyl-1-thia-2,7-diazaspiro[4.4]nonane 1,1-dioxide (268EDL017 - E197)

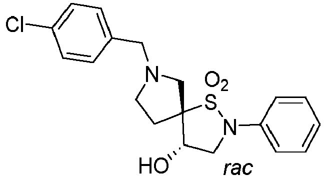

**UPLC-MS (m/z): 393.1-395.2 [M+H]⁺**. ¹H NMR (500 MHz, DMSO-*d*₆) δ 7.41-7.33 (m, 6H), 7.20 (dt, *J* = 7.9, 1.1 Hz, 2H), 7.12-7.08 (m, 1H), 6.23 (bs, 1H), 4.37 (t, *J* = 6.8 Hz, 1H), 3.80 (dd, *J* = 9.0, 6.4 Hz, 1H), 3.64 (d, *J* = 13.4 Hz, 1H), 3.58 (d, *J* = 13.4 Hz, 1H), 3.42 (dd, *J* = 9.0, 7.0 Hz, 1H), 3.24 (d, *J* = 10.8 Hz, 1H), 2.84 (td, *J* = 8.2, 4.2 Hz, 1H), 2.63 (d, *J* = 10.8 Hz, 1H), 2.45 (m, 1H), 2.43-2.34 (m, 1H), 2.21 (dt, *J* = 13.6, 7.5 Hz, 1H).

### rac-(4S,5R)-7-(3,4-dichlorobenzyl)-4-hydroxy-2-phenyl-1-thia-2,7-diazaspiro[4.4]nonane 1,1-dioxide (268EDL022 - E202)

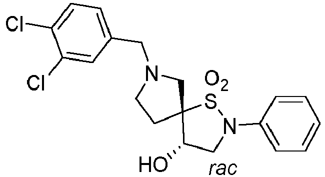

**UPLC-MS** (m/z): **427.1-429.1 [M+H]⁺**. ¹H NMR (500 MHz, DMSO-*d*₆) δ 7.62-7.56 (m, 2H), 7.40-7.30 (m, 3H), 7.23-7.19 (m, 2H), 7.11 (tt, *J* = 7.3, 1.1 Hz, 1H), 6.23 (bs, 1H), 4.38 (t, *J* = 6.6 Hz, 1H), 3.80 (dd, *J* = 9.0, 6.3 Hz, 1H), 3.67 (d, *J* = 13.8 Hz, 1H), 3.61 (d, *J* = 13.8 Hz, 1H), 3.43 (dd, *J* = 9.0, 7.0 Hz, 1H), 3.25 (d, *J* = 10.8 Hz, 1H), 2.85 (td, *J* = 8.2, 4.2 Hz, 1H), 2.65 (d, *J* = 10.8 Hz, 1H), 2.47 (m, 1H), 2.40 (ddd, *J* = 13.6, 7.8, 4.3 Hz, 1H), 2.22 (dt, *J* = 13.5, 7.6 Hz, 1H).

### rac-(4S,5R)-4-hydroxy-2-phenyl-7-(quinolin-3-ylmethyl)-1-thia-2,7-diazaspiro[4.4]nonane 1,1-dioxide (268EDL023 - E203)

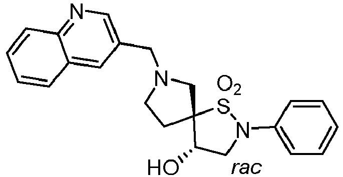

**UPLC-MS (m/z): 410.2 [M+H]⁺**. ¹H NMR (500 MHz, DMSO-*d*₆) δ 8.88 (d, *J* = 2.1 Hz, 1H), 8.25 (d, *J* = 1.4 Hz, 1H), 8.01 (d, *J* = 8.4 Hz, 1H), 7.96 (dd, *J* = 8.3, 1.1 Hz, 1H), 7.74 (ddd, *J* = 8.4, 6.9, 1.5 Hz, 1H), 7.61 (ddd, *J* = 8.1, 6.8, 1.2 Hz, 1H), 7.41-7.31 (m, 2H), 7.23-7.17 (m, 2H), 7.10 (tt, *J* = 7.5, 1.1 Hz, 1H), 6.25 (s, 1H), 4.38 (t, *J* = 6.6 Hz, 1H), 3.87 (d, *J* = 13.5 Hz, 1H), 3.83 (d, *J* = 13.5 Hz, 1H), 3.79 (dd, *J* = 9.0, 6.2 Hz, 1H), 3.43 (dd, *J* = 9.0, 7.0 Hz, 1H), 3.28 (d, *J* = 10.9 Hz, 1H), 2.90 (td, *J* = 8.1, 4.6 Hz, 1H), 2.73 (d, *J* = 10.9 Hz, 1H), 2.58 (q, *J* = 7.6 Hz, 1H), 2.43 (ddd, *J* = 12.3, 7.6, 4.6 Hz, 1H), 2.29 - 2.19 (m, 1H).

### rac-(4S,5R)-4-hydroxy-7-((1-methyl-1H-indol-3-yl)methyl)-2-phenyl-1-thia-2,7-diazaspiro[4.4]nonane 1,1-dioxide (268EDL024- E204)

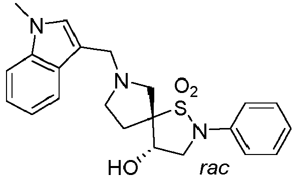

**UPLC-MS (m/z): 412.2 [M+H]⁺**. ¹H NMR (500 MHz, DMSO-*d*₆) δ 7.64 (dt, *J* = 7.9, 1.0 Hz, 1H), 7.40-7.32 (m, 3H), 7.24 (s, 1H), 7.21-7.17 (m, 2H), 7.14 (ddd, *J* = 8.2, 7.0, 1.2 Hz, 1H), 7.09 (tt, *J* = 7.4, 1.1 Hz, 1H), 7.02 (ddd, *J* = 8.0, 7.0, 1.0 Hz, 1H), 6.20 (bs, 1H), 4.35 (m, 1H), 3.78 (dd, *J* = 9.1, 6.4 Hz, 1H), 3.73 (s, 5H), 3.41 (dd, *J* = 9.0, 7.0 Hz, 1H), 3.27 (d, *J* = 10.9 Hz, 1H), 2.83 (td, *J* = 8.2, 4.4 Hz, 1H), 2.69 (d, *J* = 10.8 Hz, 1H), 2.50 (m, 1H), 2.36 (ddd, *J* = 13.6, 7.6, 4.4 Hz, 1H), 2.18 (dt, *J* = 13.5, 7.5 Hz, 1H).

### rac-(4S,5R)-7-(benzofuran-2-ylmethyl)-4-hydroxy-2-phenyl-1-thia-2,7-diazaspiro[4.4]nonane 1,1-dioxide (268EDL025 - E205)

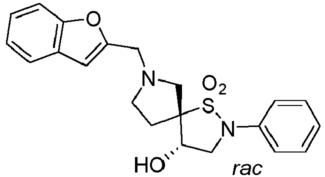

**UPLC-MS (m/z): 399.2 [M+H]⁺**. ¹H NMR (500 MHz, DMSO-*d*₆) δ 7.63-7.57 (m, 1H), 7.54 (dd, *J* = 8.1, 0.9 Hz, 1H), 7.36 (m, 2H), 7.30-7.25 (m, 1H), 7.24-7.18 (m, 3H), 7.14-7.07 (m, 1H), 6.80 (s, 1H), 6.26 (bs, 1H), 4.39 (t, *J* = 6.7 Hz, 1H), 3.82 (s, 2H), 3.78-3.82 (m, 1H), 3.43 (dd, *J* = 9.0, 7.1 Hz, 1H), 3.37 (d, *J* = 10.8 Hz, 1H), 2.91 (dt, *J* = 8.1, 4.1 Hz, 1H), 2.80 (d, *J* = 10.9 Hz, 1H), 2.60 (q, *J* = 7.6 Hz, 1H), 2.40 (ddd, *J* = 13.6, 7.6, 4.6 Hz, 1H), 2.24-2.16 (m, 1H).

### rac-(4S,5R)-7-((2,3-dihydro-1H-inden-5-yl)methyl)-4-hydroxy-2-phenyl-1-thia-2,7-diazaspiro[4.4]nonane 1,1-dioxide (268EDL030 - E210)

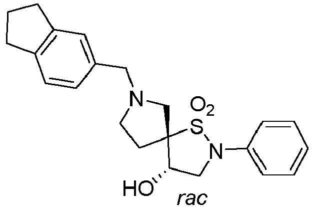

**UPLC-MS (m/z): 399.2 [M+H]⁺**. ¹H NMR (500 MHz, DMSO-*d*₆) δ 7.40-7.32 (m, 2H), 7.22-7.17 (m, 2H), 7.17-7.14 (m, 2H), 7.13-7.08 (m, 1H), 7.06 (d, *J* = 7.7 Hz, 1H), 6.22 (bs, 1H), 4.36 (t, *J* = 6.7 Hz, 1H), 3.79 (dd, *J* = 9.0, 6.3 Hz, 1H), 3.60 (d, *J* = 12.9 Hz, 1H), 3.52 (d, *J* = 12.9 Hz, 1H), 3.42 (dd, *J* = 9.0, 7.1 Hz, 1H), 3.24 (d, *J* = 10.8 Hz, 1H), 2.83 (m, 5H), 2.62 (d, *J* = 10.9 Hz, 1H), 2.45 (q, *J* = 7.6 Hz, 1H), 2.38 (ddd, *J* = 12.0, 7.6, 4.2 Hz, 1H), 2.19 (dt, *J* = 13.4, 7.5 Hz, 1H), 2.00 (quint, *J* = 7.4 Hz, 2H).

### rac-(4S,5R)-7-(3-fluoro-4-methoxybenzyl)-4-hydroxy-2-phenyl-1-thia-2,7-diazaspiro[4.4]nonane 1,1-dioxide (268EDL031 - E211)

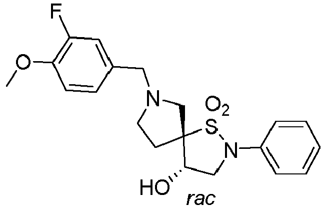

### 6.3 Synthesis of compounds E226, E228

Typical procedure for reductive amination

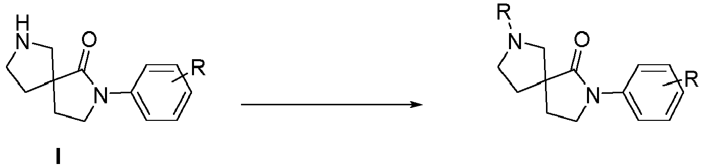

A suspension of 2-(4-methoxy-3-methylphenyl)-2,7-diazaspiro[4.4]nonan-1-one or 2-(3-fluorophenyl)-2,7-diazaspiro[4.4]nonan-1-one l (0.21 mmol) and the aldehyde (0.23 mmol, 1.1 eq) in THF (1 mL) was stirred at room temperature for 30 minutes. Sodium triacetoxyborohydride (0.47 mmol, 2.2 eq) was then added and the resulting mixture was stirred at room temperature overnight. The medium was treated with an aqueous solution of sodium bicarbonate, the aqueous layer was extracted with EA, the organic layer dried over sodium sulphate, filtered and the filtrate was concentrated under reduced pressure. Purification of the residue by preparative RP-HPLC (Puriflash) afforded desired compound.

### 7-(3-chlorobenzyl)-2-(3-fluorophenyl)-2,7-diazaspiro[4.4]nonan-1-one (268EDL051 - E226)

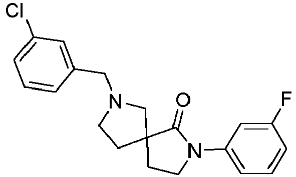

**UPLC-MS (m/z): 359.2-361.2 [M+H]⁺**. ¹H NMR (500 MHz, Chloroform-d) δ 7.56 (dt, *J* = 11.5, 2.3 Hz, 1H), 7.39-7.33 (m, 2H), 7.30 (td, *J* = 8.2, 6.5 Hz, 1H), 7.26-7.18 (m, 3H), 6.83 (tdd, *J* = 8.2, 2.5, 1.0 Hz, 1H), 3.79-3.65 (m, 3H), 3.60 (d, *J* = 13.2 Hz, 1H), 2.97 (td, *J* = 8.3, 3.9 Hz, 1H), 2.77 (d, *J* = 9.2 Hz, 1H), 2.64 (d, *J* = 9.1 Hz, 1H), 2.56 (q, *J* = 8.3 Hz, 1H), 2.38 (ddd, *J* = 12.3, 8.3, 3.9 Hz, 1H), 2.29 (ddd, *J* = 12.5, 7.3, 5.1 Hz, 1H), 2.15 (dt, *J* = 12.6, 7.2 Hz, 1H), 1.83 (dt, *J* = 12.7, 7.8 Hz, 1H).

### 7-(3-chlorobenzyl)-2-(4-methoxy-3-methylphenyl)-2,7-diazaspiro[4.4]nonan-1-one (268EDL053 - E228)

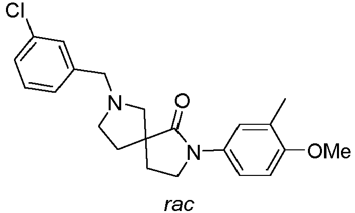

**UPLC-MS (m/z): 385.3-387.3 [M+H]⁺**. ¹H NMR (500 MHz, Chloroform-d) δ 7.42 (dd, *J* = 2.8, 0.9 Hz, 1H), 7.39-7.32 (m, 2H), 7.24-7.22 (m, 3H), 6.80 (d, *J* = 8.8 Hz, 1H), 3.81 (s, 3H), 3.75-3.64 (m, 3H), 3.60 (d, *J* = 13.2, Hz, 1H), 2.97 (td, *J* = 8.3, 3.7 Hz, 1H), 2.77 (d, *J* = 9.1 Hz, 1H), 2.64 (d, *J* = 9.1 Hz, 1H), 2.55 (q, *J* = 8.4 Hz, 1H), 2.37 (ddd, *J* = 12.3, 8.3, 3.7 Hz, 1H), 2.27 (ddd, *J* = 12.5, 7.1, 5.3 Hz, 1H), 2.22 (s, 3H), 2.13 (dt, *J* = 12.5, 7.3 Hz, 1H), 1.82 (dt, *J* = 12.6, 7.8 Hz, 1H).

### REFERENCES

- Howard C. Ansel, Nicholas Popovich and Lloyd Allen Jr ; "Pharmaceutical Dosage Forms and Drug Delivery Systems"; Lippincott Williams and Wilkins; 6th Revised edition (1 Dec. 1994) • ISBN-10: 0683001930 • ISBN-13: 978-0683001938 ; 11th edition edition (1 Nov. 2017) • ISBN-10: 1496347285 • ISBN-13: 978-1496347282.
- Cappariello A, Maurizi A, Veeriah V, Teti A. The Great Beauty of the osteoclast. Arch Biochem Biophys. 2014 Sep 15;558:70-8. doi: 10.1016/j.abb.2014.06.017.
- Loyd V. Allen, jr, Nicholas G Popovich and Howard C. Ansel. Ansel's Pharmaceutical Dosage Forms and Drug Delivery Systems. 2005. 8th Edition. Baltimore, Md: Lippincott Williams & Wilkins. ASIN: B0028IEHHY
- Vives V, Laurin M, Cres G, Larrousse P, Morichaud Z, Noel D, Côté JF, Blangy A. The Rac1 exchange factor Dock5 is essential for bone resorption by osteoclasts. J Bone Miner Res. 2011 May;26(5):1099-110. doi: 10.1002/jbmr.282.
- Vives V, Cres G, Richard C, Busson M, Ferrandez Y, Planson AG, Zeghouf M, Cherfils J, Malaval L, Blangy A. Pharmacological inhibition of Dock5 prevents osteolysis by affecting osteoclast podosome organization while preserving bone formation, Nat Commun. 2015 Feb 3;6:6218. doi: 10.1038/ncomms7218.

## Claims

1. A compound of formula (I): 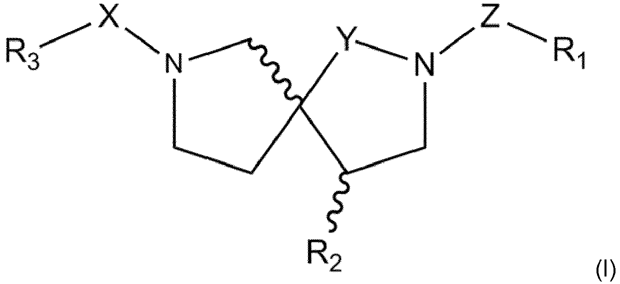 or a pharmaceutically acceptable salt thereof or solvate or stereoisomer or a mixture of stereoisomers, wherein
X is -CH₂-, -CH₂-CH₂-, -C(O)-, -C(O) -CH₂-, -SO₂-, -C(O)NH-, -OC(O)NH-, -C(O)O-, or a single bond;
Y is -C(O)- or -S(O)₂-;
Z is -CH₂- or a single bond;
R₁ is a saturated, unsaturated or aromatic 5- or 6-membered carbocycle or heterocycle, optionally substituted with one to three substituents independently selected from the group consisting of halogen, C₁-C₆ alkyl, C₁-C₆ alkoxy, NH(C₁-C₆ alkyl), N(C₁-C₆ alkyl)₂, C₁-C₆ alcohol;
R₂ is H or -OR₄; wherein R₄ is a -C₁-C₆ alkyl group optionally substituted by -OH, -OR, - COOH, -COOR, -CONHR or -CONRR' with R and R' independently representing a C₁-C₆ alkyl group; or wherein R₄ is an aromatic 5- to 7-membered carbocycle or heterocycle; R₃ is:
• H or a C₁-C₆ alkyl group, provided that when R₃ is H, X is not -C(O)- or -SO₂- ; or
• a saturated or unsaturated 3- to 7-membered carbocycle or heterocycle, optionally substituted with one to three substituents independently selected from the group consisting of halogen, C₁-C₆ alkyl and C₁-C₆ alkoxy group; or
• an aromatic 5- or 10-membered carbocycle or heterocycle, optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, and a C₁-C₆ alkyl wherein 1 to 3 hydrogen atoms are replaced by a fluorine atom, S(O)₂R, COOH, COOR, CONHR or CONRR' with R and R' independently representing a C₁-C₆ alkyl group.

2. The compound of claim 1, wherein R₁ is a cyclohexyl group or a phenyl group, optionally substituted with one or two substituents independently selected from the group consisting of halogen, C₁-C₆ alkyl, and C₁-C₆ alkoxy group; advantageously R₁ is a cyclohexyl group or a phenyl group, optionally substituted with one or two substituents independently selected from the group consisting of halogen and methoxy group; advantageously R₁ is cyclohexyl, phenyl, 3-chlorophenyl, 3-fluorophenyl, 3-methyl-4-methoxyphenyl, more advantageously R₁ is phenyl.

3. The compound of claim 1 or claim 2, wherein R₂ is H or -OR₄; wherein R₄ is H or a C₁-C₆ alkyl group substituted by -OH, -OR, -COOH, -COOR, -CONHR or -CONRR'; or a aromatic 5- to 7-membered heterocycle; preferably R₄ is H or a C₁-C₆ alkyl group substituted by -OH, -OR, -COOR or -CONHR; or a pyridinyl; more preferably R₄ is H; with R and R' independently representing a C₁-C₆ alkyl group.

4. The compound of any one of claims 1 to 3, wherein R and R' are independently methyl or ethyl.

5. The compound of any of claims 1 to 4, wherein R₃ is
• H or CH₃
• a saturated 5- to 7-membered carbocycle, optionally substituted with one to three substituents independently selected from the group consisting of halogen, C₁-C₆ alkyl and C₁-C₆ alkoxy group, or
• a phenyl, naphthyl, indanyl, indenyl, furanyl, pyrrolyl, thienyl, oxazolyl, isoxazolinyl, thiazolyl, isothiazolyl, imidazolyl, pyrazolyl, pyridinyl, pyrimidinyl, pyrazinyl, pyridazinyl, indolyl, thiophenyl, benzofuranyl, benzothiophenyl, 1,3-benzodioxolyl, quinoleyl, and isoquibnoleyl group, each group being optionally substituted with one to three substituents independently selected from the group consisting of: halogen, cyano, hydroxy, C₁-C₆ alkyl, C₁-C₆ alkoxy group, NH(C₁-C₆ alkyl), N(C₁-C₆ alkyl)₂, C₁-C₆ alcohol, -CO₂CH₃, and a C₁-C₆ alkyl wherein 1 to 3 hydrogen atoms are replaced by a fluorine atom.

6. The compound of any one of claims 1 to 4, wherein R₃ is:
• H or CH₃
• a cyclopentyl or cyclohexyl group, or
• a phenyl, naphthyl, indanyl, isoxazolinyl, imidazolyl, pyrazolyl, thienyl, pyridinyl, indolyl, thiophenyl, benzofuranyl, 1,3-benzodioxolyl, or quinoleyl group, preferably a phenyl, indanyl, benzofuranyl, 1,3-benzodioxolyl, or quinoleyl group, each group being optionally substituted with one to three substituents independently selected from the group consisting of: : halogen, cyano, hydroxy, C₁-C₆ alkyl, C₁-C₆ alkoxy group, NH(C₁-C₆ alkyl), N(C₁-C₆ alkyl)₂, C₁-C₆ alcohol, -CO₂CH₃, and a C₁-C₆ alkyl wherein 1 to 3 hydrogen atoms are replaced by a fluorine atom.

7. The compound of any one of claims 1 to 4, wherein R₃ is selected from the group consisting of:
| | | | |
|---|---|---|---|
| 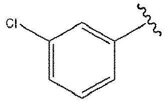 | 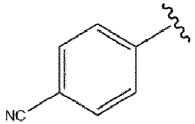 | 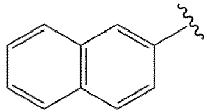 | 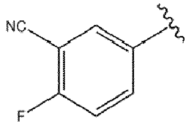 |
| 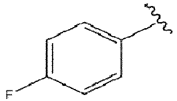 | 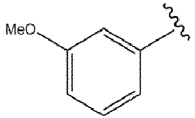 | 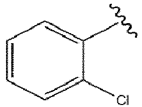 | 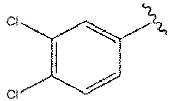 |
| 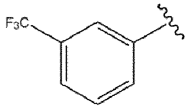 | 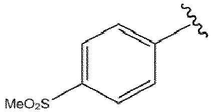 | 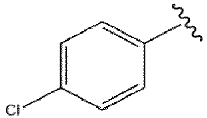 | 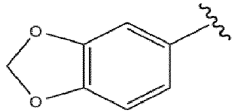 |
| 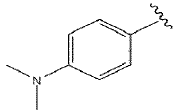 | 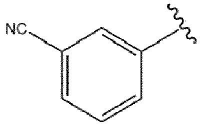 | 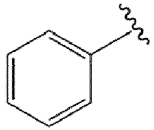 | 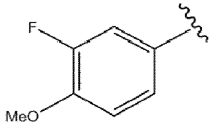 |
| 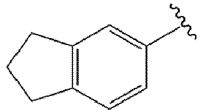 | 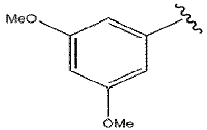 | 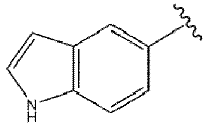 | 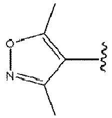 |
| 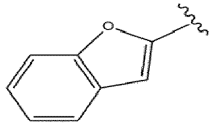 | 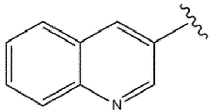 | 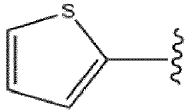 | 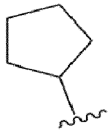 |
| 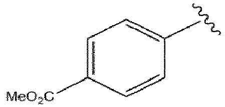 | 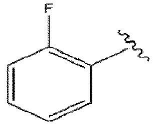 | 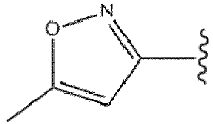 | 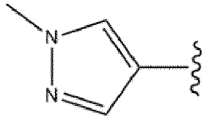 |
| 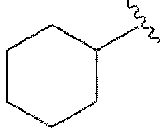 | 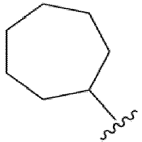 | 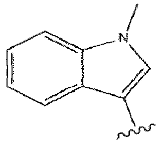 | H |
| CH₃ | | | |

8. The compound of claim 7, wherein X is CH₂, R₁ is phenyl group, optionally substituted with one or two substituents independently selected from the group consisting of halogen and methoxy; and R₂ is H.

9. The compound of any one of claims 1 to 8, wherein Y is SO₂.

10. The compound of claim 1, wherein it is selected from the group consisting of: 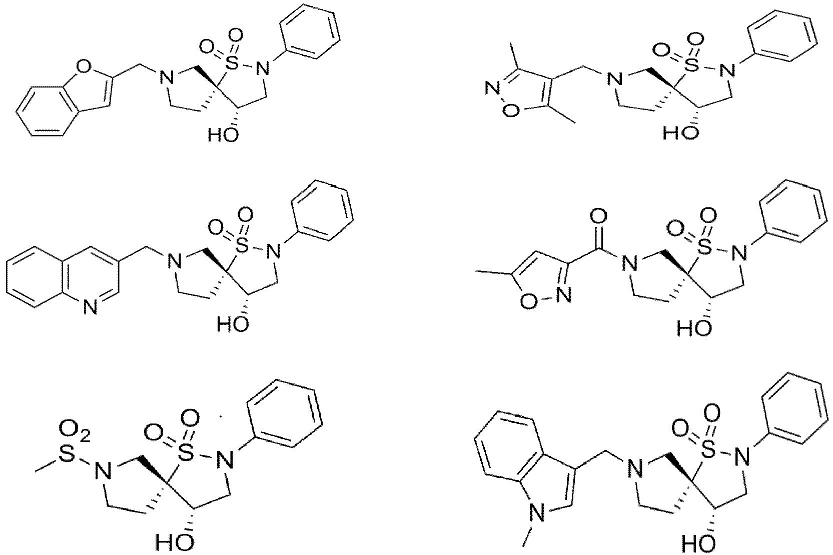 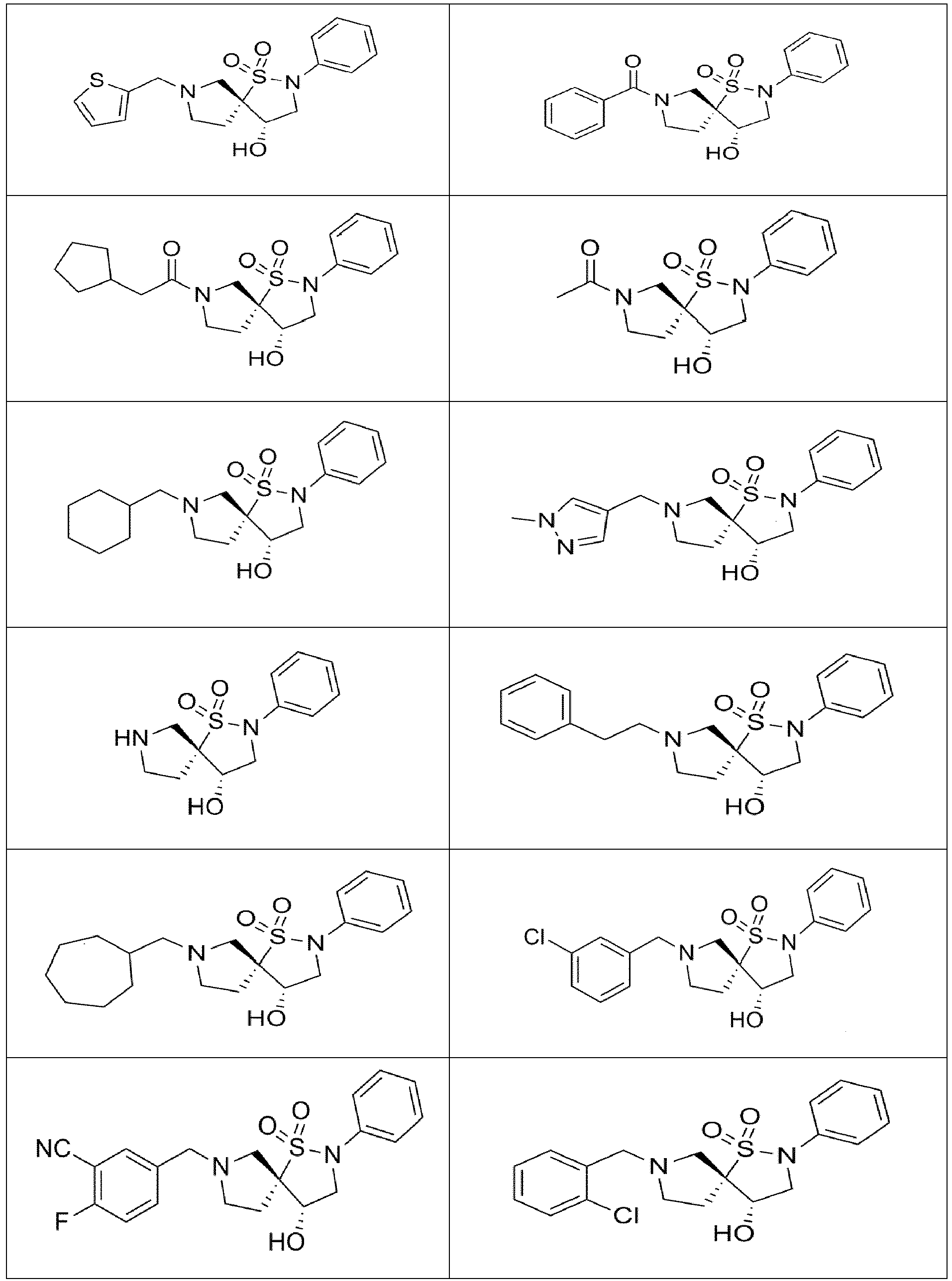 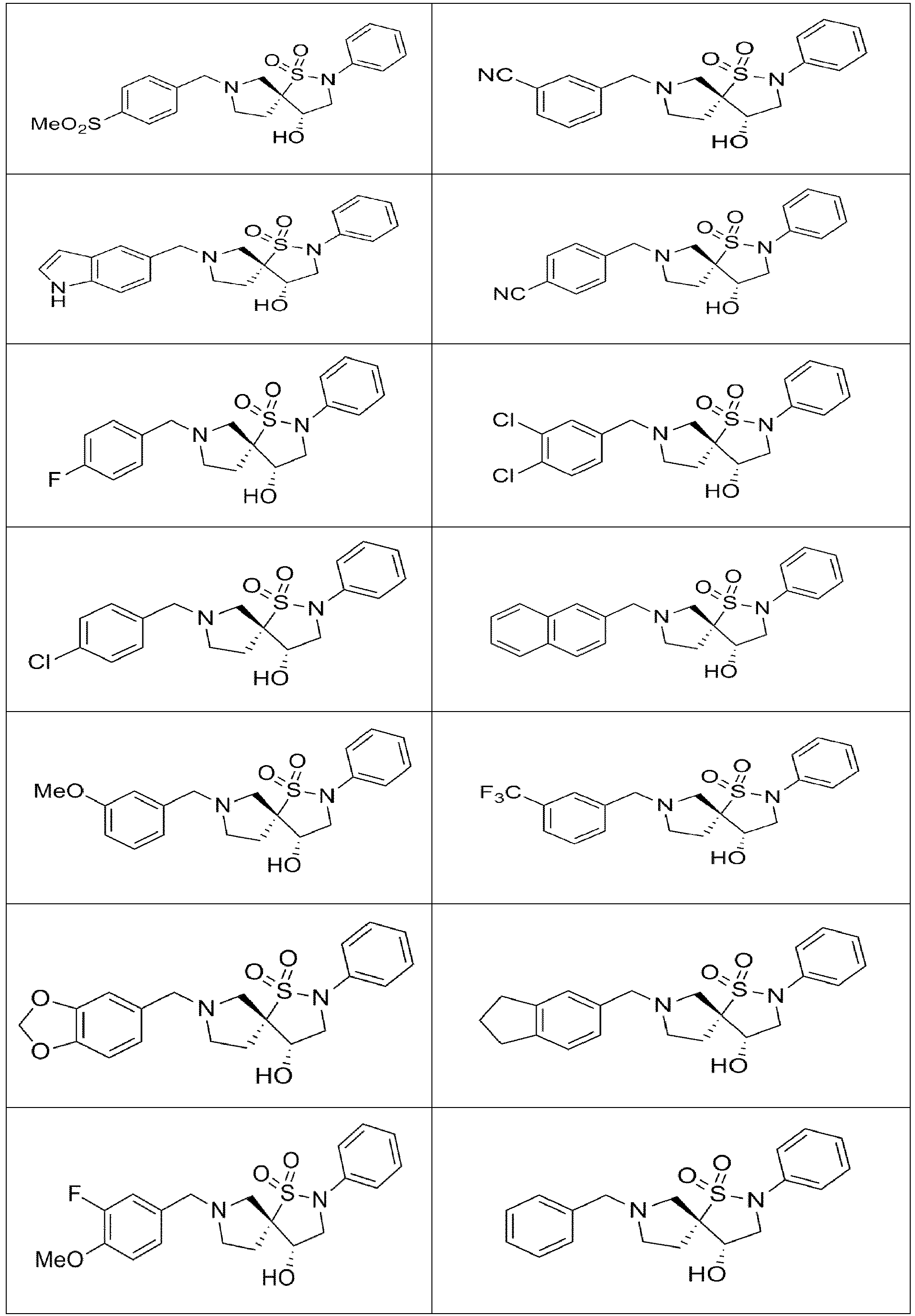 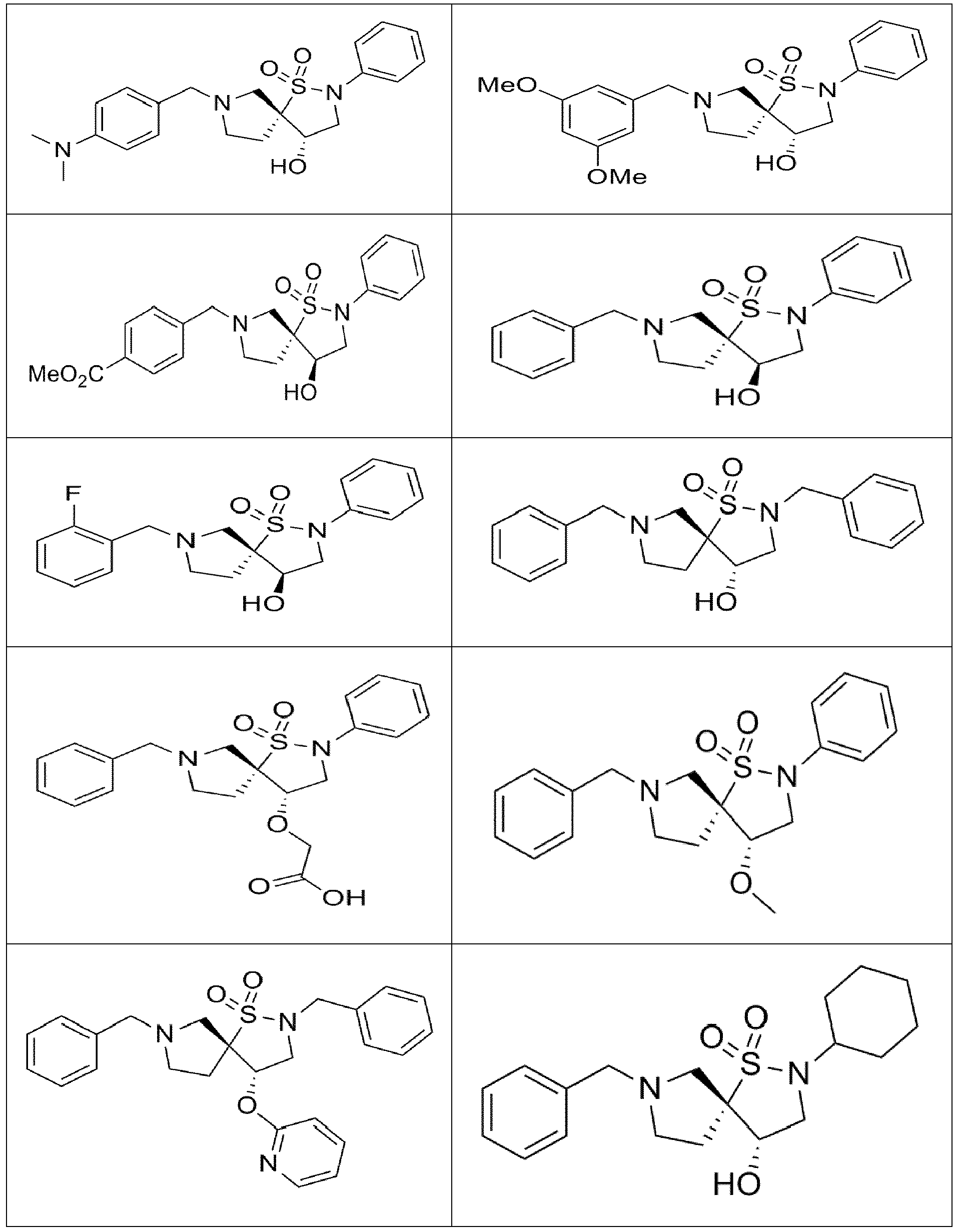 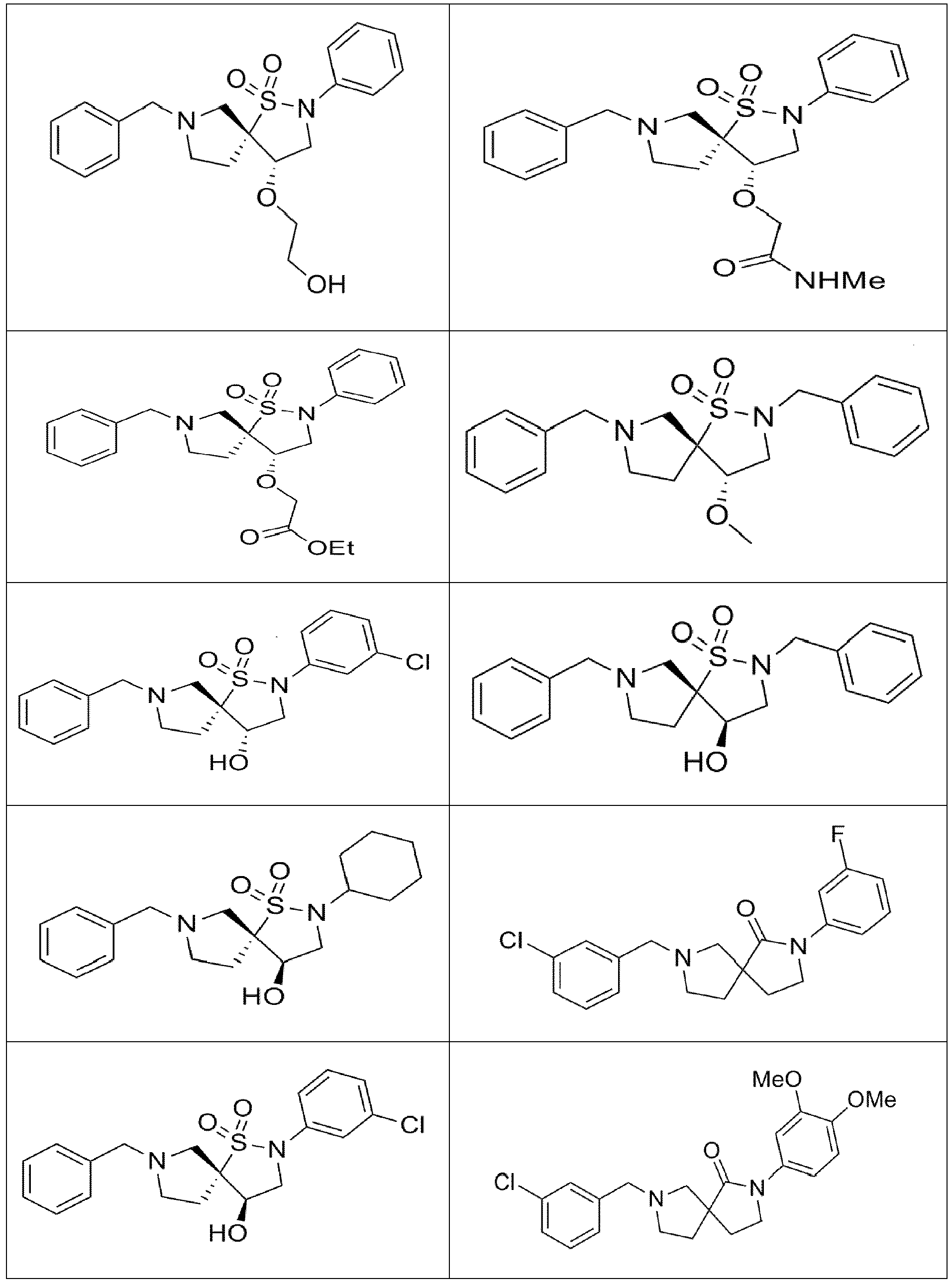 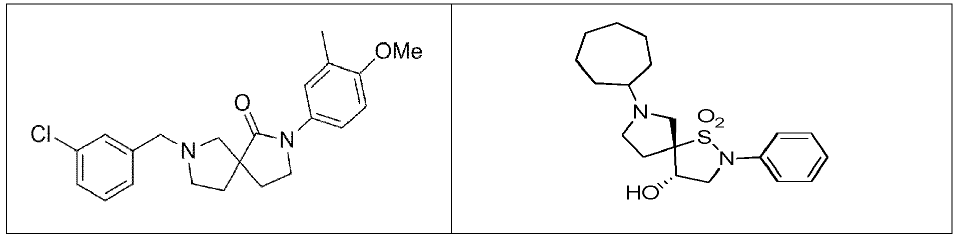 or a pharmaceutically acceptable salt and/or solvate thereof.

11. A pharmaceutical composition comprising a compound of any of claims 1 to 10 as active ingredient, and a pharmaceutically acceptable excipient.

12. The compound of any of claims 1 to 10 or the composition of claim 11, for use as drug.

13. The compound of any of claims 1 to 10 or the composition of claim 11, for use in prevention and/or treatment of disorder-associated bone loss or disease-associated bone loss, preferably selected in the group consisting of menopause, osteoporosis, osteopenia due to bone metastasis, osteogenesis imperfecta, inflammatory arthritis, particularly rheumatoid arthritis, more particularly periarticular erosions in rheumatoid arthritis, primary hyperparathyroidism, hypercalcemia of malignancy, Paget's disease of bone, periodontal disease, immobilization induced osteopenia, and glucocorticoid treatment.

14. The compound of any of claims 1 to 10 or the composition of claim 11, for use in prevention and/or treatment of bone loss caused by cancer, particularly multiple myeloma, and bone metastases, particularly bone metastases associated with cancer.

15. The compound of any of claims 1 to 10 or the composition of claim 11 for use according to claims 14 or 15, wherein the subject in need thereof is a mammal, preferably selected from rodent, cat, dog, primate, equine and human.
